# EUROPEAN PATENT APPLICATION

(11) **EP 4 205 756 A1**
(43) Date of publication of application: **05.07.2023**
(21) Application number: 21862142.3
(22) Date of filing: 30.08.2021
(51) Int. Cl.: A61K 36/634, A61P 13/08, A61P 17/14, A61K 8/9789, A61Q 7/00

(54) **PHARMACEUTICAL COMPOSITION COMPRISING ABELIOPHYLLUM DISTICHUM EXTRACT AS ACTIVE INGREDIENT FOR TREATING ANDROGEN RECEPTOR-RELATED DISEASE**

(30) Priority: 31.08.2020 KR 20200109845; 27.08.2021 KR 20210114027
(71) Applicant: Konkuk University Glocal Industry-Academic Collaboration Foundation, Chungju-si, Chungcheongbuk-do 27478 (KR)
(72) Inventor: LEE, Seung Hyun, Seoul 05229 (KR); KIM, Eun-Kyung, Chungju-si Chungcheongbuk-do 27369 (KR); MOON, Sang Ho, Chungju-si Chungcheongbuk-do 27353 (KR); CHOI, Young-Jin, Hwaseong-si Gyeonggi-do 18366 (KR)
(74) Representative: Dehns
(86) International application number: PCT/KR2021/011564
(87) International publication number: WO 2022/045846

(57) **Abstract**

The present invention relates to a pharmaceutical composition comprising an Abeliophyllum distichum extract as an active ingredient for treating androgen receptor-related diseases. The *Abeliophyllum distichum* extract according to the present invention or isoquercetin has an inhibitory activity against the androgen receptor and an excellent therapeutic effect on benign prostatic hyperlasia animal models, thus this finding can apply widely to the industry for relevant therapeutic agents for androgen receptor-related diseases such as benign prostatic hyperplasia, androgen-dependent hair loss, dysuria, and prostate cancer.

## Description

### [Technical Field]

The present invention relates to a pharmaceutical composition for treating an androgenic receptor-related disease, which includes an *Abeliophyllum distichum* extract as an active ingredient.

This application claims priority to and the benefits of Korean Patent Application No. 10-2020-0109845, filed on August 31, 2020, and Korean Patent Application No. 10-2021-0114027, filed on August 27, 2021, the disclosure of which is incorporated herein by reference in their entirety.

### [Background Art]

Androgen is a generic term for hormones that affect the growth and development of the male reproductive system, and also refers to all materials that exhibit the action of male hormones. Androgens are hormones that act on the development of male secondary sexual characteristics and are mainly secreted from male testes, and some are also secreted from the adrenal cortex and female ovaries. Androgens are 19-carbon steroids, and include dihydrotestosterone generated by reduction in cells, androsterone or dehydroepiandrosterone, which is changed therefrom and secreted into urine, and adrenosterone secreted from the adrenal cortex, as well as testosterone secreted from testes.

They control the development, maintenance, and function of the reproductive organs and other sexual characteristics. Particularly, they are involved in an increase in proteins in bone tissue, an increase in the weight and size of the kidneys, an increase in activity of sweat and sebaceous glands, and generation of red blood cells. When they are applied to the skin, sebum increases due to the thickened stratum corneum of the epidermis, and they are also the cause of acne occurring in adolescents. In addition, an androgen is associated with body hair, and as the androgen increases, male facial hair tends to increase. However, androgens can also promote hair loss, which is the loss of hair on the forehead or crown.

Therefore, among androgen-dependent diseases caused by an abnormal increase in androgens, benign prostatic hyperplasia (BPH) was defined in the past as a condition in which the flow of urine is reduced due to obstruction of the urethra by blocking the passage of urine from the lower bladder due to an enlarged prostate, and also defined histologically as the proliferation of epithelial tissue cells of the prostate stromal cells or prostate. However, since the pathophysiology of the disease is too complex to explain with such definitions or concepts, currently, in men aged 50 years or older, BPH is defined as a complaint of lower urinary tract symptoms, divided into storage symptoms including frequent urination (urinating 8 times a day or more), nocturia, urinary urgency in which there is a strong and sudden urge to urinate (a feeling of needing to urinate) and an inability to resist the urge, and symptoms indicating bladder emptying problems, including urinary hesitancy (urination onset delay), intermittency (not continuous), and straining to empty the bladder.

As major clinical characteristics, there are prostate enlargement and lower urinary tract symptoms. It is known that prostate enlargement occurs in the presence of androgens, and anabolic steroids increase a prostate capacity and decrease a urine flow, resulting in increased urinary frequency.

The prostate is an androgen-dependent organ in which testosterone and its extratesticular origin are activated into more potent dihydrotestosterone (DHT). Typically, the prostate is an organ critical for DHT formation, and the systemic effect of the endocrine activity of the prostate is mainly involved in DHT formation and its excretion for circulation. DHT production increases with age, and causes increased prostate growth and hypertrophy. The importance of DHT is confirmed by clinical studies in which a 5α-reductase inhibitor is applied to BPH male patients. In many cases, it is known that a therapeutic method using the 5α-reductase inhibitor significantly reduces a DHT level of the prostate and a prostate size. Finasteride is widely used in treatment of androgen-dependent diseases, e.g., male pattern baldness, BPH, and prostate cancer. Finasteride is a competitive, specific inhibitor of 5α-reductase, and blocks the conversion of testosterone into DHT in the prostate, hair follicles and other androgen-sensitive tissue, causing the inhibition of a DHT concentration in serum and the prostate. Conventionally used drugs such as finasteride and dutasteride proved to be therapeutically effective for BPH, but their use is strictly limited due to side effects such as erectile dysfunction, loss of libido, dizziness and upper respiratory tract infection.

Meanwhile, Korean Abelia (scientific name: *Abeliophyllum distichum*) is a tree belonging to the ash family, and is the only species belonging to the genus *Abeliophyllum.* It is endemic to the Korean peninsula and grows sparsely at the foot of sunny mountains in Gyeonggi-do and Chungcheong-do.

However, there have been no reports on a composition for preventing, alleviating or treating androgenic receptor-related diseases including an *Abeliophyllum distichum* extract as an active ingredient.

### [Disclosure]

### [Technical Problem]

Therefore, the present inventors confirmed that an *Abeliophyllum distichum* extract and its active ingredient isoquercetin have anti-androgen activity, inhibit androgen signaling and related factors, and have a significant effect on a BPH animal model. Thus, the present invention was completed.

Accordingly, the present invention is directed to providing a pharmaceutical composition, which includes isoquercetin or an *Abeliophyllum distichum* extract as an active ingredient, for preventing or treating an androgenic receptor-related disease.

The present invention is directed to providing a food composition, which includes isoquercetin or an *Abeliophyllum distichum* extract as an active ingredient, for preventing or alleviating an androgenic receptor-related disease.

However, technical problems to be solved in the present invention are not limited to the above-described problems, and other problems which are not described herein will be fully understood by those of ordinary skill in the art from the following descriptions.

### [Technical Solution]

To achieve the purpose of the present invention, the present invention provides a pharmaceutical composition, which includes isoquercetin or an *Abeliophyllum distichum* extract as an active ingredient, for preventing or treating an androgenic receptor-related disease.

The present invention also provides a food composition, which includes isoquercetin or an *Abeliophyllum distichum* extract as an active ingredient, for preventing or alleviating an androgenic receptor-related disease.

In one embodiment of the present invention, the extract may be an extract prepared with one or more solvents selected from the group consisting of water, C₁ to C₆ alcohols, acetone, ether, benzene, chloroform, ethyl acetate, methylene chloride, hexane, cyclohexane, petroleum ether, a subcritical fluid, and a supercritical fluid, but the present invention is not limited thereto.

In another embodiment of the present invention, the androgenic receptor-related disease may be one or more selected from the group consisting of prostate cancer, BPH, dysuria and hair loss, but the present invention is not limited thereto.

In still another embodiment of the present invention, the *Abeliophyllum distichum* extract may include isoquercetin, but the present invention is not limited thereto.

In yet another embodiment of the present invention, the *Abeliophyllum distichum* extract may be an *Abeliophyllum distichum* leaf extract, but the present invention is not limited thereto.

In yet another embodiment of the present invention, the composition may inhibit the expression of an androgenic receptor, but the present invention is not limited thereto.

In yet another embodiment of the present invention, the composition may inhibit one or more androgen signaling-related factors selected from the group consisting of 5α-reductase 2 (5AR2), steroid receptor coactivator 1 (SRC1), an estrogen receptor (ER), and a prostate-specific antigen (PSA), but the present invention is not limited thereto.

In yet another embodiment of the present invention, the composition may inhibit a PI3K/AKT signaling pathway, but the present invention is not limited thereto.

In yet another embodiment of the present invention, the food composition may be a health functional food, but the present invention is not limited thereto.

In addition, the present invention provides a method of preventing or treating an androgenic receptor-related disease, which includes administering a composition including isoquercetin or an *Abeliophyllum distichum* extract as an active ingredient to a subject.

In addition, the present invention provides a use of a composition including isoquercetin or an *Abeliophyllum distichum* extract as an active ingredient for preventing or treating an androgenic receptor-related disease.

In addition, the present invention provides a use of isoquercetin or an *Abeliophyllum distichum* extract for producing a drug for preventing or treating an androgenic receptor-related disease.

### [Advantageous Effects]

Since an *Abeliophyllum distichum* extract or isoquercetin according to the present invention has androgenic receptor-inhibitory activity, and has an excellent therapeutic effect on a benign prostatic hyperplasia (BPH) animal model, it can be widely used in industries associated with androgenic receptor-related diseases, including BPH, androgen-dependent hair loss, dysuria and prostate cancer.

### [Description of Drawings]

FIGS. 1A and 1B show the inhibitory effects on the overexpression of testosterone (TP)-induced AR, 5AR2, and PSAby treatment of prostatic cells (LNCaP) with an *Abeliophyllum distichum* leaf (ADL) hot water (D. W.) extract, an ADL ethanol (EtOH) extract, and an ADL hexane extract.
FIG. 2 shows the inhibitory effects on the overexpression of testosterone (TP)-induced AR, 5AR2, and PSA by treatment of prostatic cells (LNCaP) with an ADL hot water (ADLD) extract, an ADL ethanol (ADLE) extract, and an ADL hexane (ADLH) extract, confirmed by fluorescence staining.
FIG. 3 shows the inhibitory effects on the overexpression of testosterone (TP)-induced AR, 5AR2, and PSA according to the time for harvesting ADLs.
FIG. 4 shows a chromatogram for analyzing a metabolite of an ADLE extract.
FIGS. 5A and 5B show the inhibitory effects of four types of *Abeliophyllum distichum* functional ingredients (isoquercetin (IQ), rutin, chlorogenic acid (CA), and isorhamnetin 3-glucoside-7-rhamnoside (IR)) on the overexpression of testosterone (TP)-induced AR, 5AR2, and PSA.
FIGS. 6A and 6B show the inhibitory effects on the overexpression of testosterone (TP)-induced AR, 5AR2, PSA and PI3K by a concentration of isoquercetin (IQ) (positive control: Fi, finasteride).
FIGS. 7A and 7B show the inhibitory effects on testosterone (TP)-induced PI3K (Cell Signaling), PCNA (SantaCruz) and cyclin D1 (Cell Signaling) according to concentrations of isoquercetin (IQ) and an ADL extract.
FIG. 8 shows the result of analyzing a content of isoquercetin according to the time of extracting *Abeliophyllum distichum.*
FIG. 9 shows the process of manufacturing a benign prostatic hyperplasia (BPH) animal model, and the administration plan for an *Abeliophyllum distichum* extract (positive controls: Saw palmetto (saw) or Finasteride (Fi)).
FIGS. 10A to 10E show the results of measuring prostate indexes after oral administration of 100 mg/kg/d of an *Abeliophyllum distichum* extract to BPH animal models, wherein FIG. 10A shows a prostate size, FIG. 10B shows a prostate weight, FIG. 10C shows a ratio of a prostate weight with respect to a body weight, FIG. 10D shows a lumen area of prostate tissue, and FIG. 10E shows an epithelial thickness of prostate tissue.
FIG. 11A shows the expression levels of 5AR2, SCR1, AR, ER, and PSA in prostate tissues obtained after oral administration of 100 mg/kg/d of an *Abeliophyllum distichum* extract to BPH animal models.
FIG. 11B shows the quantitative result of analyzing the expression levels of 5AR2, SCR1, AR, ER, and PSA in prostate tissues after oral administration of 100 mg/kg/d of an *Abeliophyllum distichum* extract to BPH animal models.
FIG. 11C shows the result of H&E staining for observing the prostatic epithelial cells in prostate tissue obtained after oral administration of 100 mg/kg/d of an *Abeliophyllum distichum* extract to BPH animal models.
FIG. 11D shows the result of measuring DHT levels in serum obtained after oral administration of 100 mg/kg/d of an *Abeliophyllum distichum* extract to BPH animal models.
FIG. 12A shows the result of measuring the expression levels of PI3K, p-AKT, and t-AKT in prostate tissue obtained after oral administration of 100 mg/kg/d of an *Abeliophyllum distichum* extract to BPH animal models.
FIG. 12B shows the quantitative result of analyzing the expression levels of PI3K, p-AKT, and t-AKT in prostate tissue obtained after oral administration of 100 mg/kg/d of an *Abeliophyllum distichum* extract to BPH animal models.
FIG. 13a shows the result of measuring the expression levels of EGF (SantaCruz), IGF-1 (ABCam), TGF-1β (SantaCruz), and VEGF (SantaCruz) in prostate tissue obtained after oral administration of 100 mg/kg/d of an *Abeliophyllum distichum* extract to BPH animal models.
FIG. 13B shows the quantitative result of analyzing the expression levels of EGF (SantaCruz), IGF-1 (ABCam), TGF-1β (SantaCruz), and VEGF (SantaCruz) in prostate tissue obtained after oral administration of 100 mg/kg/d of an *Abeliophyllum distichum* extract to BPH animal models.
FIG. 14A shows the result of measuring the expression levels of PCNA and cyclin D1 in prostate tissue obtained after oral administration of 100 mg/kg/d of an *Abeliophyllum distichum* extract to BPH animal models.
FIG. 14B shows the quantitative result of analyzing the expression levels of PCNA and cyclin D1 in prostate tissue obtained after oral administration of 100 mg/kg/d of an *Abeliophyllum distichum* extract to BPH animal models.
FIG. 14C shows the histochemical staining result for observing PCNA in prostate tissue obtained after oral administration of 100 mg/kg/d of an *Abeliophyllum distichum* extract to BPH animal models.
FIG. 15A shows the result of measuring the expression levels of Bax (SantaCruz) and Bcl-2 (Cell Signaling) in prostate tissue obtained after oral administration of 100 mg/kg/d of an *Abeliophyllum distichum* extract to BPH animal models.
FIG. 15B shows the quantitative result of analyzing the expression levels of Bax (SantaCruz) and Bcl-2 (Cell Signaling) in prostate tissue obtained after oral administration of 100 mg/kg/d of an *Abeliophyllum distichum* extract to BPH animal models.
FIG. 16 shows the result of measuring prostate indexes after oral administration of 1 mg/kg or 10 mg/kg of isoquercetin (IQ) to BPH animal models (A: prostate size, B: prostate weight, C: a ratio of prostate weight to body weight).
FIG. 17 shows the inhibitory effects on the expression of AR, SRC-1, and 5AR2 in prostate tissue obtained after oral administration of 1 mg/kg or 10 mg/kg of isoquercetin (IQ) to BPH animal models.

### [Best modes of the Invention]

The present invention provides a pharmaceutical composition, which includes isoquercetin or an *Abeliophyllum distichum* extract as an active ingredient, for preventing or treating an androgenic receptor-related disease.

Hereinafter, the present invention will be described in detail.

The name *"Abeliophyllum distichum"* used herein is more preferably *Abeliophyllum distichum* Nakai, but the present invention is not limited thereto. *Abeliophyllum distichum* is a tree belonging to the ash family, and is the only species belonging to the genus *Abeliophyllum.* It is endemic to the Korean peninsula and grows sparsely at the foot of sunny mountains in Gyeonggi-do and Chungcheong-do.

In the present invention, *Abeliophyllum distichum* may be used without damaging its original form, or may be used after a pretreatment process in consideration of a process speed and process (manufacturing) efficiency intended by one of ordinary skill in the art, and the pretreatment process may include, for example, normal screening, washing with water, cutting, powdering, and drying.

In the present invention, *Abeliophyllum distichum* is not limited to its harvest period, but may be harvested in spring or autumn, preferably autumn, and more preferably late autumn.

An *Abeliophyllum distichum* extract used herein may be obtained from all or a part of the plant, for example, one or more selected from the group consisting of stems, roots, leaves, fruits, and flowers. In one embodiment of the present invention, an *Abeliophyllum distichum* extract was obtained from its leaves.

The *Abeliophyllum distichum* extract of the present invention may be extracted by a known method of extracting a natural material. For example, the *Abeliophyllum distichum* extract of the present invention may be extracted with one or more selected from the group consisting of water, C₁ to C₆ organic solvents, and subcritical or supercritical fluids. The C₁ to C₆ organic solvents may be one or more selected from the group consisting of C₁ to C₆ alcohols, acetones, ethers, benzenes, chloroform, ethyl acetate, methylene chloride, hexane, cyclohexane, and petroleum ether, but the present invention is not limited thereto.

The *Abeliophyllum distichum* extract of the present invention is preferably extracted with ethanol, but the present invention is not limited thereto. As an extraction method, conventional extraction methods such as macerating, digesting, and heating using the solvent can be used.

In the present invention, the ethanol concentration is not limited, and for example, the *Abeliophyllum distichum* extract of the present invention may be a 30 to 100%, 40 to 100%, 50 to 100%, 60 to 100%, 50 to 90%, 60 to 90%, 60 to 80%, 65 to 75%, or approximately 70% ethanol extract.

In the present invention, isoquercetin is also known by the IUPAC name 3-(β-D-glucopyranosyloxy)-3',4',5,7-tetrahydroxyflavone, is a compound represented by C₂₁H₂₀O₁₂, and having a molecular weight of 464.379 [g/mol], and is registered under CAS registration No. 482-35-9. The isoquercetin may be represented by Formula 1 below.

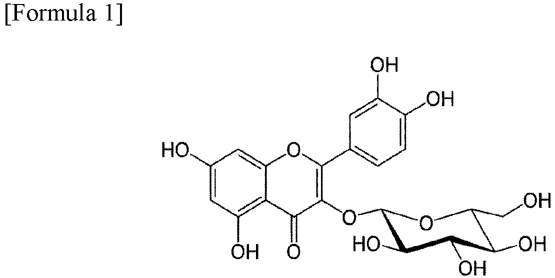

The composition of the present invention may inhibit the expression of an androgenic receptor, but the present invention is not limited thereto.

The composition of the present invention may inhibit one or more androgen signaling-related factors selected from the group consisting of 5α-reductase 2 (5AR2), steroid receptor coactivator 1 (SRC1), estrogen receptor (ER), and prostate-specific antigen (PSA), but the present invention is not limited thereto.

The composition of the present invention may inhibit the PI3K/AKT signaling pathway, but the present invention is not limited thereto.

In one embodiment of the present invention, as a result of confirming the expression levels of androgen signaling-related factors such as AR, 5AR2, and PSA according to a solvent for an *Abeliophyllum distichum* extract, it was confirmed that an *Abeliophyllum distichum* ethanol extract has the most excellent inhibitory effect (see Example 2).

In one embodiment of the present invention, as a result of confirming the expression levels of androgen signaling-related factors such as AR, SAR2, and PSA according to the harvest period of *Abeliophyllum distichum* leaves, it was confirmed that the inhibitory effect of an extract of *Abeliophyllum distichum* harvested in late autumn was superior to that of an extract of *Abeliophyllum distichum* harvested in late spring (see Example 4).

In one embodiment of the present invention, as a result of confirming the expression levels of androgen signaling-related factors AR, 5AR2, and PSA according to the treatment of isoquercetin, which is one of the functional ingredients of an *Abeliophyllum distichum* leaf extract, it was confirmed that isoquercetin has significant AR, 5AR2, and PSA inhibitory effects (see Example 5-2).

In one embodiment of the present invention, as a result of confirming the downregulation effect on the expression of PI3K, and cell proliferation factors, proliferating cell nuclear antigen (PCNA) and cyclin D1 according to isoquercetin treatment, it was confirmed that the expression of PI3K, PCNA, and cyclin D1 is significantly reduced depending on the concentrations of isoquercetin and an *Abeliophyllum distichum* leaf ethanol extract (see Example 5-3).

In one embodiment of the present invention, as a result of confirming the BPH treatment effects of an *Abeliophyllum distichum* extract and isoquercetin in BPH animal models, it was confirmed that the *Abeliophyllum distichum* extract and isoquercetin significantly improve prostate indexes, and significantly improve the levels of an androgenic receptor and prostate-related factors (see Example 7).

Therefore, the *Abeliophyllum distichum* extract or isoquercetin of the present invention may be used as an active ingredient of a composition for treating an androgenic receptor-related disease.

The term "androgenic receptor-related disease" used herein refers to various diseases caused by signal transmission that can occur due to abnormal, excessive stimulation of an androgenic receptor (AR), and the diseases may include, but are not particularly limited to, prostate cancer, BPH, dysuria and hair loss.

The normal development and preservation of the prostate depend on the role of the male hormones, or androgens, through an androgenic receptor. In the normal prostate, 1 to 2% of cells per day die and are replaced by new cells, which is part of normal prostatic function regulated by an androgenic receptor. Therefore, maintaining the normal function of an androgenic receptor is the key to prostate health. Particularly, prostate cancer cells mainly depend on an androgenic receptor for growth and survival, and are essential for both androgen-dependent prostate cancer and androgen-independent prostate cancer. Accordingly, a therapy for inhibiting the transcription action of an androgenic receptor plays an important role in treatment of prostate cancer.

In addition, the biggest trigger for the occurrence of BPH is androgenic hormone (AH) that affects the growth and development of the male reproductive system, and the androgenic hormone binds to an androgenic receptor (AR) to cause the enlargement of the prostate. Therefore, in BPH treatment, research is being conducted focusing on the downregulation of the expression of an androgenic receptor.

Meanwhile, an androgenic receptor also has an important relationship with hair loss. Testosterone encounters 5-α-reductase and is converted to dihydrotestosterone (DHT). Excessively formed DHT binds to an androgenic receptor in hair follicles, causing hair loss. Androgenic hormone (AH) acts only via an androgenic receptor, and the action mechanisms of testosterone and dihydrotestosterone depend on binding to an androgenic receptor. Therefore, to prevent hair loss, the downregulation of androgenic receptor expression in hair follicles is important.

Therefore, the *Abeliophyllum distichum* extract or isoquercetin according to the present invention may downregulate the expression of an androgenic receptor at the transcription level (mRNA level), so it may be effectively used in treatment of androgenic receptor-related diseases, such as prostate cancer, BPH, dysuria, and hair loss.

In the present invention, isoquercetin may be included in the form of a pharmaceutically acceptable salt as an active ingredient. The term "pharmaceutically acceptable salt" includes salts derived from pharmaceutically acceptable inorganic acids, organic acids, or bases.

Examples of suitable acids may include hydrochloric acid, bromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, citric acid, methanesulfonic acid, formic acid, benzoic acid, malonic acid, gluconic acid, naphthalene-2-sulfonic acid, and benzenesulfonic acid. An acid-addition salt may be prepared by a conventional method, for example, by dissolving a compound in an excess of an aqueous acid solution and precipitating the salt using a water-miscible organic solvent such as methanol, ethanol, acetone or acetonitrile. In addition, an acid-addition salt may be prepared by heating equimolar amounts of a compound and an acid or alcohol in water, and then dehydrating the mixture through evaporation or suction filtering a precipitated salt.

Salts derived from suitable bases may include alkali metals such as sodium and potassium, alkaline earth metals such as magnesium, and ammonium, but the present invention is not limited thereto. The alkali metal or alkaline earth metal may be obtained by, for example, dissolving a compound in an excess of an alkali metal hydroxide or alkaline earth metal hydroxide solution, filtering a non-dissolved compound salt, and then evaporating and drying the filtrate. Here, as a metal salt, particularly, preparing a sodium, potassium or calcium salt is suitable for a pharmaceutical purpose, and a silver salt corresponding thereto may be obtained by reacting an alkali metal or alkaline earth metal salt with a suitable silver salt (e.g., silver nitrate).

A content of the *Abeliophyllum distichum* extract or isoquercetin in the composition of the present invention may be suitably adjusted according to symptoms of a disease, the progression of symptoms, or the condition of a patient, and may be, for example, 0.0001 to 99.9 wt%, or 0.001 to 50 wt% based on the total weight of the composition, but the present invention is not limited thereto. The content ratio is a value based on the dry content after removing a solvent.

The pharmaceutical composition according to the present invention may further include a suitable carrier, excipient and diluent, which are conventionally used in the preparation of a pharmaceutical composition. The excipient may be, for example, one or more selected from the group consisting of a diluent, a binder, a disintegrant, a lubricant, an adsorbent, a humectant, a film-coating material, and a controlled-release additive.

The pharmaceutical composition according to the present invention may be formulated in the form of a powder, a granule, a sustained-release granule, an enteric granule, a liquid, an ophthalmic solution, an elixir, an emulsion, a suspension, a spirit, a troche, aromatic water, a lemonade, a tablet, a sustained-release tablet, an enteric tablet, a sublingual tablet, a hard capsule, a soft capsule, a sustained-release capsule, an enteric capsule, a pill, a tincture, a soft extract, a dry extract, a fluid extract, an injection, a capsule, a perfusate, a plaster, a lotion, a paste, a spray, an inhalant, a patch, a sterile injection, or an external preparation such as an aerosol according to a conventional method, and the external preparation may be formulated in a cream, a gel, a patch, a spray, an ointment, a plaster, a lotion, a liniment, a paste or a cataplasma.

The carrier, excipient and diluent which may be included in the pharmaceutical composition according to the present invention may include lactose, dextrose, sucrose, an oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and mineral oil.

The pharmaceutical composition may be formulated with a diluent or an excipient such as a filler, a thickening agent, a binder, a wetting agent, a disintegrant, and a surfactant, which are commonly used.

As additives for a tablet, powder, granule, capsule, pill and troche, excipients such as corn starch, potato starch, wheat starch, lactose, sucrose, glucose, fructose, dimannitol, precipitated calcium carbonate, synthetic aluminum silicate, calcium monohydrogen phosphate, calcium sulfate, sodium chloride, sodium bicarbonate, purified lanolin, microcrystalline cellulose, dextrin, sodium alginate, methylcellulose, carboxymethylcellulose, sodium carboxymethylcellulose, kaolin, urea, colloidal silica gel, hydroxypropyl starch, hydroxypropyl methyl cellulose, HPMC 1928, HPMC 2208, HPMC 2906, HPMC 2910, propylene glycol, casein, calcium lactate and Primojel; binders such as gelatin, gum arabic, ethanol, agar powder, cellulose acetate phthalate, carboxymethyl cellulose, carboxymethyl cellulose calcium, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch powder, hydroxypropylcellulose, hydroxypropylmethylcellulose, polyvinyl alcohol and polyvinylpyrrolidone; disintegrants such as hydroxypropylmethylcellulose, corn starch, agar powder, methylcellulose, bentonite, hydroxypropyl starch, sodium carboxymethylcellulose, calcium citrate, sodium lauryl sulfate, silicic anhydride, 1-hydroxypropyl cellulose, dextran, an ion exchange resin, polyvinyl acetate, formaldehyde-treated casein and gelatin, alginic acid, amylose, guar gum, sodium bicarbonate, polyvinylpyrrolidone, calcium phosphate, gelled starch, gum arabic, amylopectin, pectin, sodium polyphosphate, ethyl cellulose, sucrose, magnesium aluminum silicate, a di-sorbitol solution and light anhydrous silicic acid; and lubricants such as calcium stearate, magnesium stearate, stearic acid, hydrogenated vegetable oil, talc, limestone kaolin, petrolatum, sodium stearate, cacao butter, sodium salicylate, magnesium salicylate, polyethylene glycol 4000 and 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, a higher fatty acid, a higher alcohol, silicone oil, paraffin oil, polyethylene glycol fatty acid ether, starch, sodium chloride, sodium acetate, sodium oleate, dileucine and light anhydrous silicic acid may be used.

Additives for a liquid according to the present invention may be water, diluted hydrochloric acid, diluted sulfuric acid, sodium citrate, monostearate sucrose, polyoxyethylene sorbitol fatty acid esters (Tween esters), polyoxyethylene monoalkylethers, lanolin ethers, lanolin esters, acetic acid, hydrochloric acid, acetic acid, hydrochloric acid, aqueous ammonia, ammonium carbonate, potassium hydroxide, sodium hydroxide, prolamine, polyvinylpyrrolidone, ethyl cellulose, and sodium carboxymethylcellulose.

In a syrup according to the present invention, a solution of white sugar, other sugars, or a sweetener may be used, and if necessary, a flavoring agent, a colorant, a preservative, a stabilizer, a suspending agent, an emulsifier, or a viscosity-controlling agent may be used.

In an emulsion according to the present invention, distilled water may be used, and if necessary, an emulsifier, a preservative, a stabilizer, or a flavoring agent may be used.

In a suspension according to the present invention, a suspending agent such as acacia, tragacanth, methyl cellulose, carboxymethyl cellulose, sodium carboxymethyl cellulose, sodium carboxymethyl cellulose, microcrystalline cellulose, sodium alginate, hydroxypropylmethylcellulose (HPMC), HPMC 1828, HPMC 2906, or HPMC 2910 may be used, and if necessary, a surfactant, a preservative, a stabilizer, a coloring agent, or a flavoring agent may be used.

In an injection according to the present invention, a solvent such as injectable sterile water, 0.9% sodium chloride for injection, Ringer's solution, dextrose for injection, dextrose+sodium chloride for injection, PEG, lactated Ringer's solution, ethanol, propylene glycol, non-volatile oil-sesame oil, cottonseed oil, peanut oil, soybean oil, corn oil, ethyl oleate, isopropyl myristic acid or benzene benzoate; a solubilizing agent such as sodium benzoate, sodium salicylate, sodium acetate, urea, urethane, monoethylacetamine, butazolidine, propylene glycol, Tween, nicotinamide, hexamine or dimethylacetamide; a buffer such as a weak acid and a salt thereof (acetic acid and sodium acetate), a weak base and a salt thereof (ammonia and ammonium acetate), an organic compound, a protein, albumin, peptone, or gums; an isotonic agent such as sodium chloride; a stabilizer such as sodium bisulfite (NaHSO₃), carbon dioxide gas, sodium metabisulfite (Na₂S₂O₃), sodium sulfite (Na₂SO₃), nitrogen gas (N₂) or ethylenediaminetetracetic acid; an antioxidant such as sodium bisulfide 0.1%, sodium formaldehyde sulfoxylate, thiourea, disodium ethylenediaminetetraacetate or acetone sodium bisulfite; a pain-relief agent such as benzyl alcohol, chlorobutanol, procaine hydrochloride, glucose or calcium gluconate; or a suspending agent such as sodium CMC, sodium alginate, Tween 80 or aluminum monostearate may be included.

In a suppository according to the present invention, a base such as cacao butter, lanolin, Witepsol, polyethylene glycol, glycerogelatin, methyl cellulose, carboxymethylcellulose, a mixture of stearate and oleate, Subanal, cottonseed oil, peanut oil, palm oil, cacao butter + cholesterol, lecithin, Lanette wax, glycerol monostearate, Tween or Span, Imhausen, monolene (propylene glycol monostearate), glycerin, Adeps solidus, Buytyrum Tego-G, Cebes Pharma 16, hexalide base 95, Cotomar, Hydrokote SP, S-70-XXA, S-70-XX75 (S-70-XX95), Hydrokote 25, Hydrokote 711, Idropostal, Massa estrarium, A, AS, B, C, D, E, I, T), Mass-MF, Masupol, Masupol-15, neosuppostal-N, paramount-B, supposiro (OSI, OSIX, A, B, C, D, H, L), suppository base IV types (AB, B, A, BC, BBG, E, BGF, C, D, 299), Suppostal (N, Es), Wecoby (W, R, S, M ,Fs), or a Tegester triglyeride base (TG-95, MA, 57) may be may be used.

A solid formulation for oral administration may be a tablet, a pill, a powder, a granule or a capsule, and such a solid formulation may be prepared by mixing at least one excipient, for example, starch, calcium carbonate, sucrose, lactose or gelatin, with the active ingredient. Also, in addition to the simple excipient, lubricants such as magnesium stearate and talc may also be used.

As a liquid formulation for oral administration, a suspension, a liquid for internal use, an emulsion, or a syrup may be used, and a generally-used simple diluent such as water or liquid paraffin, as well as various types of excipients, for example, a wetting agent, a sweetener, a fragrance and a preservative, may be included. A formulation for parenteral administration may be a sterilized aqueous solution, a non-aqueous solvent, a suspension, an emulsion, a lyophilizing agent or a suppository. As the non-aqueous solvent or suspension, propylene glycol, polyethylene glycol, a vegetable oil such as olive oil, or an injectable ester such as ethyl oleate may be used.

The composition according to the present invention is administered at a pharmaceutically effective amount. In the present invention, "pharmaceutically effective amount" used herein refers to an amount sufficient for treating a disease at a reasonable benefit/risk ratio applicable for medical treatment, and an effective dosage may be determined by parameters including a type of a patient's disease, severity, drug activity, sensitivity to a drug, administration time, an administration route and an excretion rate, the duration of treatment and drugs simultaneously used, and other parameters well known in the medical field.

The pharmaceutical composition of the present invention may be administered separately or in combination with other therapeutic agents, and may be sequentially or simultaneously administered with a conventional therapeutic agent, or administered in a single or multiple dose(s). In consideration of all of the above-mentioned parameters, it is important to achieve the maximum effect with the minimum dose without side effects, and such a dose may be easily determined by one of ordinary skill in the art.

The pharmaceutical composition of the present invention may be administered into a subject via various routes. All administration routes may be expected, and the pharmaceutical composition of the present invention may be administered by, for example, oral administration, subcutaneous injection, intraperitoneal administration, intravenous, intramuscular or intrathecal injection, sublingual administration, buccal administration, rectal insertion, vaginal insertion, ocular administration, ear administration, nasal administration, inhalation, spraying through the mouth or nose, skin administration, or transdermal administration.

The pharmaceutical composition of the present invention may be determined according to the type of a drug that is an active ingredient, as well as several related factors including a disease to be treated, an administration route, a patient's age, sex and body weight, and the severity of the disease.

The term "subject" used herein refers to a target in need of treatment, and more specifically, a mammal such as a human or a non-human primate, a mouse, a rat, a dog, a cat, a horse, or a cow, but the present invention is not limited thereto.

"Administration" used herein refers to providing the composition of the present invention to a subject by any suitable method.

The term "prevention" used herein refers to all actions of inhibiting or delaying the occurrence of a targeted disease, and "treatment" used herein refers to all actions involved in improving or beneficially changing symptoms of a targeted disease or metabolic abnormalities thereby through the administration of the pharmaceutical composition according to the present invention, and "alleviation" used herein refers to all actions of reducing parameters related to a targeted disease, for example, the severity of symptoms, by the administration of the composition according to the present invention.

Moreover, the present invention provides a food composition including an *Abeliophyllum distichum* extract or isoquercetin as an active ingredient.

Examples of the food used herein include a functional food and a health functional food.

When the *Abeliophyllum distichum* extract or isoquercetin is used as a food additive, the *Abeliophyllum distichum* extract may be added alone or used in combination with another food or food component, and may be appropriately used according to a conventional method. The mixing amount of the active ingredient may be appropriately determined according to the purpose of use (prevention, health or therapeutic method). Generally, when preparing a food or beverage, the *Abeliophyllum distichum* extract of the present invention may be added at an amount of 15 wt% or less, or 10 wt% or less based on a raw material. However, in long-term consumption for health and hygiene or health control, the amount of the composition may be the same as or lower than the above-mentioned range, and since there is no problem in terms of safety, the active ingredient may be used in an amount greater than the above-mentioned range.

There is no particular limitation to the type of food. Examples of the food to which the material can be added may include meat, sausage, bread, chocolate, candy, snacks, confectionaries, pizza, ramen, other noodles, gum, dairy products including ice cream, various types of soups, beverages, tea, drinks, alcoholic beverages and vitamin complexes, and in a general sense, all health functional foods.

The health beverage composition according to the present invention may contain additional components such as various flavoring agents or natural carbohydrates like conventional beverages. The above-mentioned natural carbohydrates are monosaccharides such as glucose and fructose, disaccharides such as maltose and sucrose, polysaccharides such as dextrin and cyclodextrin, and sugar alcohols such as xylitol, sorbitol and erythritol. As the sweetener, natural sweeteners such as thaumatin and a stevia extract, or synthetic sweeteners such as saccharin and aspartame may be used. The proportion of the natural carbohydrate may generally be approximately 0.01 to 0.20 g or 0.04 to 0.10 g per 100 mL of the composition of the present invention.

In addition, the composition of the present invention may contain various nutrients, vitamins, electrolytes, flavoring agents, pectic acid and a salt thereof, alginic acid and a salt thereof, organic acids, protective colloidal thickening agents, pH adjustors, stabilizers, preservatives, glycerin, alcohols, or carbonizing agents used in carbonated beverages. Other than these, the composition of the present invention may contain fruit pulp for producing natural fruit juices, fruit drinks and vegetable drinks. Such components may be used independently or in combination. The proportion of such an additive is not very critical, but is generally selected in the range of 0.01 to 0.20 parts by weight per 100 parts by weight of the composition of the present invention.

### [Modes of the Invention]

Hereinafter, to help in understanding the present invention, exemplary examples will be suggested. However, the following examples are merely provided so that the present invention can be more easily understood, and not to limit the present invention.

### Example 1. Experimental methods

### 1-1. Preparation of hot water extract

*Abeliophyllum distichum* was provided from Uri Tree Farming Association. 100 g of *Abeliophyllum distichum* (*Abeliophyllum distichum* Nakai) leaves was added to 1000 mL of distilled water (DW) in an Erlenmeyer flask, and extracted by boiling at 100 °C for 1 hour. The extract was filtered under reduced pressure (0.2 µm pore size) and concentrated using a rotary vacuum concentrator. The resulting concentrate was then lyophilized in a freeze dryer for 5 days.

### 1-2. Preparation of 70% ethanol extract

100 g of *Abeliophyllum distichum* leaves and 1000 mL of 70% ethanol were added to an Erlenmeyer flask and extracted using a stirrer for 2 hours. The resulting extract from which *Abeliophyllum distichum* leaves were removed was then transferred to a beaker. Subsequently, 1000 mL of ethanol was added again to an Erlenmeyer flask, extracted for 2 hours, and then transferred to a beaker. The following process was performed three times. Subsequently, the 70% ethanol extract was concentrated using a rotary vacuum concentrator. The extract was then filtered under reduced pressure (0.2 µm pore size) and lyophilized in a freeze dryer for 5 days.

### 1-3. Preparation of 95% hexane extract

100 g of *Abeliophyllum distichum* leaves and 1000 mL of 95% hexane were added to an Erlenmeyer flask. The following process was the same as that in the 70% ethanol extraction.

### 1-4. Immunofluorescence

After placing slides on a 12-well plate, LNCaP cells (5×10⁴) were seeded on the slides to adhere. After 24 hours, the cells were treated with testosterone (100 nM), and then treated with 100 µg/mL each of hot water (D.W.), ethanol (EtOH), and hexane (Hexane) extracts of *Abeliophyllum distichum* leaves (*Abeliophyllum distichum* Nakai leaves, ADLs). After 24 hours, the cells were fixed in cold methanol, followed by treatment with 0.1% Triton X-100 for 1 hour. After blocking with 5% goat serum, the slides were incubated with androgenic receptor (AR) antibodies (1:300) diluted in BSA overnight at 4 °C. The slides were then treated with AlexaFluor 588 secondary antibodies (1:1,000 dilution) for 1 hour. DAPI staining was used to stain nuclei, and then coverslips were mounted. AR expression levels were confirmed using a Zeiss fluorescence microscope.

### 1-5. Western blotting

LNCaP cells were treated with testosterone propionate (TP) to induce androgenic receptor signaling, treated with *Abeliophyllum distichum* leaf hot water (D.W.), ethanol (EtOH), and hexane (Hexane) extracts, followed by confirming the efficacy of inhibiting the expression of an androgenic receptor (AR, SantaCruz), 5 alpha reductase (5AR2, ABCam), and prostate-specific antigen (PSA, SantaCruz) to compare the inhibitory effect on androgenic receptor signaling-related factors per *Abeliophyllum distichum* leaf extractLNCaP cells in LNCaP cells. The LNCaP cells (2×10⁶) were seeded in 6-well plates, and treated with 100 nM testosterone propionate (TP, Tokyo Chemical Industry) and 100 µg/mL each of *Abeliophyllum distichum* leaf hot water (D.W.), ethanol (EtOH), and hexane (Hexane) extracts for 24 hours. Here, a group treated with 1 µg/mL finasteride (Fi), which is an androgen inhibiting drug, was used as a positive control. After finishing the treatment of the cells, an androgenic receptor signaling-inhibitory effect was analyzed using western blotting. First, the cells were lysed in a protein inhibitor cocktail (Sigma)-containing radioimmunoprecipitation assay (RIPA) buffer, and a protein concentration was estimated using a BCA assay, followed by electrophoresis in a 12% SDS polyacrylamide gel. The gel was then transferred to a membrane using Mini Trans-Blot cells (Bio rad). Subsequently, the membrane was treated with primary antibodies at 4 °C for 12 hours. Subsequently, the membrane was washed with TBST for 5 minutes three times, and treated with secondary antibodies for 1 hour. Bands were then detected using an Azure c300 imaging system (Azure Biosystems) by chemiluminescence with an HPR substrate (Advansta Inc., San Jose, CA, USA). Band intensities were quantified by setting the area of each sample using ImageJ software (NIH ver. 1.48, USA) and then setting the area of corresponding β-actin in the same manner as used above, and dividing the area of β-actin by the area of each sample.

### 1-6. Manufacture of BPH models and administration plan

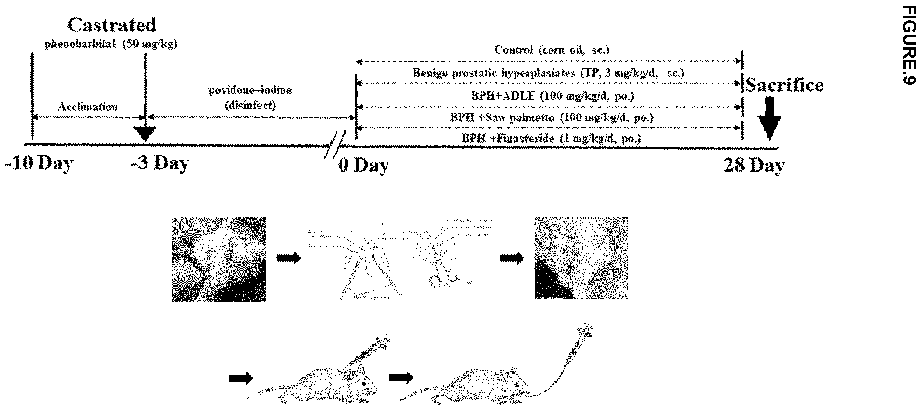

Experimental animals, 8-week-old male SD rats (weighing 200±5 g), were divided into 5 groups as shown in FIG. 9, and after 1-week acclimation, a BPH-induced group was anesthetized by intraperitoneal injection with phentobarbital (50 mg/kg), followed by excising the testis and epididymis, and then tying the surgical site using suture. All experiments were conducted in an aseptic environment. Three days after surgical recovery for the BPH-induced group (BPH), testosterone was dissolved in corn oil and then subcutaneously injected daily at 3 mg/kg, inducing BPH. For an *Abeliophyllum distichum* leaf ethanol extract-administered group (BPH+ADLE), an *Abeliophyllum distichum* leaf ethanol extract was dissolved in sterile distilled water and orally administered using a zonde at 100 mg/kg for 4 weeks. For a saw palmetto-administered group (BPH+Saw) as a positive control, saw palmetto was dissolved in sterile distilled water and orally administered at 100 mg/kg, and for a finasteride-administered group (BPH+Fi), finasteride was orally administered using a zonde at 1 mg/kg per day for 4 weeks. After the experiment, the rats were sacrificed using zoletil, blood samples were collected through heart puncture, and serum was then separated and stored at -80 °C. The prostate was separated and weighed using RNAse-free surgical tools, and stored at -80 °C, followed by western blotting.

Meanwhile, isoquercetin (IQ) administration was also performed in the same manner and using the same controls as described above, and for a low concentration-administered group (BPH+IQ1), IQ was dissolved in sterile distilled water and then orally administered at 1 mg/kg, and for a high concentration-administered group (BPH+IQ10), IQ was dissolved in sterile distilled water and then orally administered using a zonde at 10 mg/kg for 4 weeks.

### 1-7. Hematoxylin & eosin staining (H&E staining)

Prostate tissue was fixed on 10% formaldehyde and dehydrated, and then embedded in paraffin. The paraffin block was cut to 4 µm using a microtome (Leica). For H&E staining, the cut section was deparaffinated using xylene, washed, stained with hematoxylin for 5 minutes, and then washed with water for 5 minutes. Subsequently, the resulting section was stained with eosin for 30 seconds, dehydrated and then sealed. The tissue was examined using a Leica DM6 Research Inverted Phase microscope (Leica, Werzlar, Germany). The epithelial thickness and lumen area were measured using Leica Application Suite (LAS) microscope software.

### 1-8. Analysis of inhibitory effect on expression of androgen signaling-related factors

For animal experimental groups, protein expression of androgen signaling-related factors, 5α-reductase 2 (5AR2), steroid receptor coactivator 1 (SRC1), an estrogen receptor (ER), and a prostate-specific antigen (PSA), were analyzed. In the excised prostate tissue, a ventral prostate lobe part was treated with a protein inhibitor cocktail (Sigma)-containing RIPA buffer, and the prostate tissue was homogenized using a tacoPrep Bead beater. Afterward, a protein concentration was estimated using a protein BCA assay, electrophoresis was performed in a 12% SDS polyacrylamide gel, and then the gel was transferred to a membrane using Mini Trans-Blo cells (Bio rad). The membrane was then treated with primary antibodies against each protein at 4 °C for 12 hours, washed with TBST three times for 5 minutes, and treated with secondary antibodies for 1 hour. Subsequently, bands were then detected using an Azure c300 imaging system (Azure Biosystems) by chemiluminescence with an HPR substrate (Advansta Inc., San Jose, CA, USA). Band intensities were quantified by setting the area of each sample using ImageJ software (NIH ver. 1.48, USA) and then setting the area of corresponding β-actin in the same manner as used above, and dividing the area of β-actin by the area of each sample.

### 1-9. Immunohistochemistry

A4 µm-thick section was deparaffinated and washed, and the section was then immersed in a 0.01M citrate buffer (pH, 6.0) and reacted in a microwave oven for antigen retrieval. Subsequently, it was left at room temperature for 10 minutes, washed with D.W., and treated with 3% H₂O₂ for 10 minutes. It was treated with goat serum to block non-specific binding. The section was then incubated with anti-PCNA and anti-AR overnight at 4 °C. After one hour treatment with secondary antibodies, all of the immunostained sections were counter-stained with hematoxylin, followed by tissue analysis using a Leica DM6 Research Inverted Phase microscope (Leica, Werzlar, Germany).

### 1-10. Analysis of inhibitory effect of expression of PI3K/Akt signaling pathway-related factors

It was confirmed that androgen signaling was inhibited, thereby inhibiting the expression of an epithelial growth factor (EGF). Meanwhile, to confirm whether the expression of PI3K/Akt signaling pathway-related factors, phosphatidylinositol-3-kinase (PI3K) and protein kinase B (Akt), is inhibited by the *Abeliophyllum distichum* leaf ethanol extract, the above-described western blotting was performed.

### 1-11. Analysis of inhibitory effect on growth factor expression

Due to androgen signaling, growth factors are transcribed in an androgen response element (ARE), and thus their expression levels tend to increase. The growth factors may be the major cause of inducing BPH. Accordingly, to confirm whether the *Abeliophyllum distichum* leaf ethanol extract administration reduces the expression of growth factors in the prostate of a BPH-induced rat, the above-described western blotting was performed. The expression levels of the growth factors, epidermal growth factor (EGF), insulin like growth factor I (IGF-1), transforming growth factor beta 1 (TGF-1β), and vascular endothelial growth factor (VEGF) were confirmed.

### 1-12. Analysis of inhibitory effect on cell proliferation-related factors in prostate tissue

To analyze the expression of representative factors related to cell proliferation in prostate tissue, proliferating cell nuclear antigen (PCNA) and cyclin D1 protein, the above-described western blotting was performed.

### 1-13. Analysis of metabolites using UPLC-MS

Analysis of metabolites of an *Abeliophyllum distichum* leaf ethanol extract was performed using Vion UPLC^{™} system (Vion, Waters, Milford, MA, USA). LC was performed using an Acquity UPLC BEH C18 column (2.1 mm × 100 mm, 1.7 µm, Waters), and a column temperature was set to 55 °C. A flow rate was set to 0.35 mL/min. As mobile phases, 0.1% formic acid (FA)-containing water and 0.1% FA-containing acetonitrile (ACN) were used. MS operation conditions were as follows: capillary voltage - 2.5 kV, sample cone - 20 V, ion source temperature - 200 °C, desolvation temperature - 400 °C, cons gas - 30 L/h, desolvation gas - 900 L/h, scan time - 0.2 sec, scan range - m/z 50-1500, and collision energy ramp - 10-30 eV (m/z 50-1000).

### Example 2. Confirmation of expression of androgen signaling-related factors according to solvent of Abeliophyllum distichum extract

Testosterone-induced expression levels of AR, 5AR2, and PSA were identified by treating the prostate cells, LNCaP cells with the *Abeliophyllum distichum* leaf hot water, ethanol and hexane extracts prepared in Examples 1-1 to 1-3. 5AR2 is known to play important roles in development of normal prostate and enlargement of aged prostate, and as AR expression increases, it is known to bind to AR with higher DHT in the prostate and cause enlargement of the prostate. In addition, AR binds to DHT, promoting the generation of a prostate-specific antigen PSA.

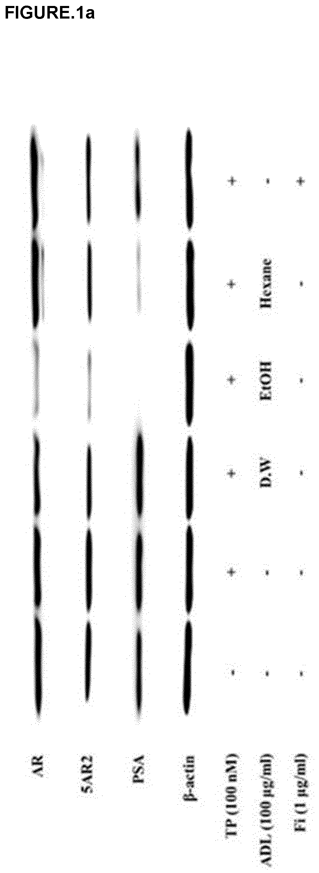

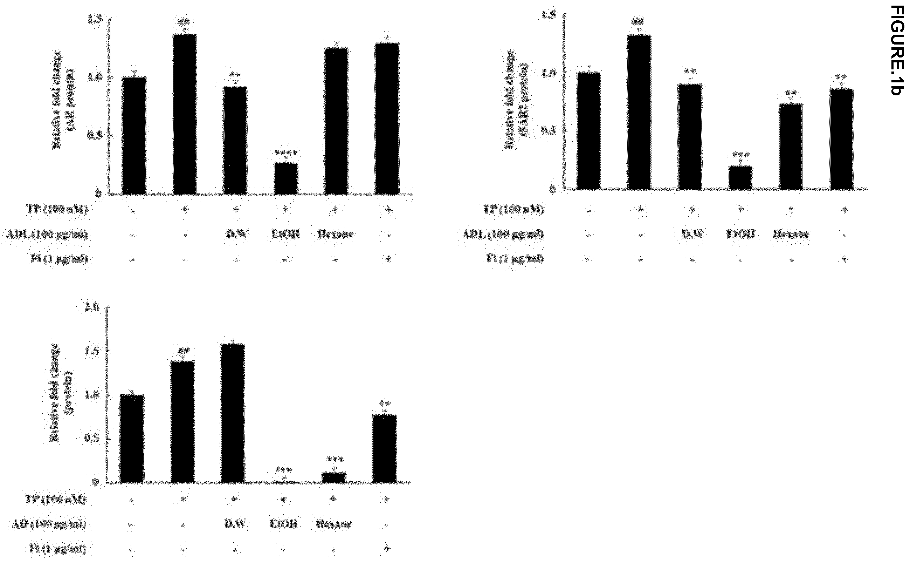

As shown in FIGS. 1A and 1B, it was confirmed that the *Abeliophyllum distichum* hot water extract significantly reduced AR and 5AR2 increased by testosterone, the hexane extract significantly reduced the expression of 5AR2 and PSA, and the ethanol extract significantly inhibited all of AR, 5AR2, and PSA and exhibited a significantly excellent inhibitory effect compared to the hot water extract and hexane extract.

### Example 3. Confirmation of expression of androgen receptors according to solvent of Abeliophyllum distichum extract

In addition to the western blotting of Example 2, an AR inhibitory effect was reconfirmed through fluorescence staining.

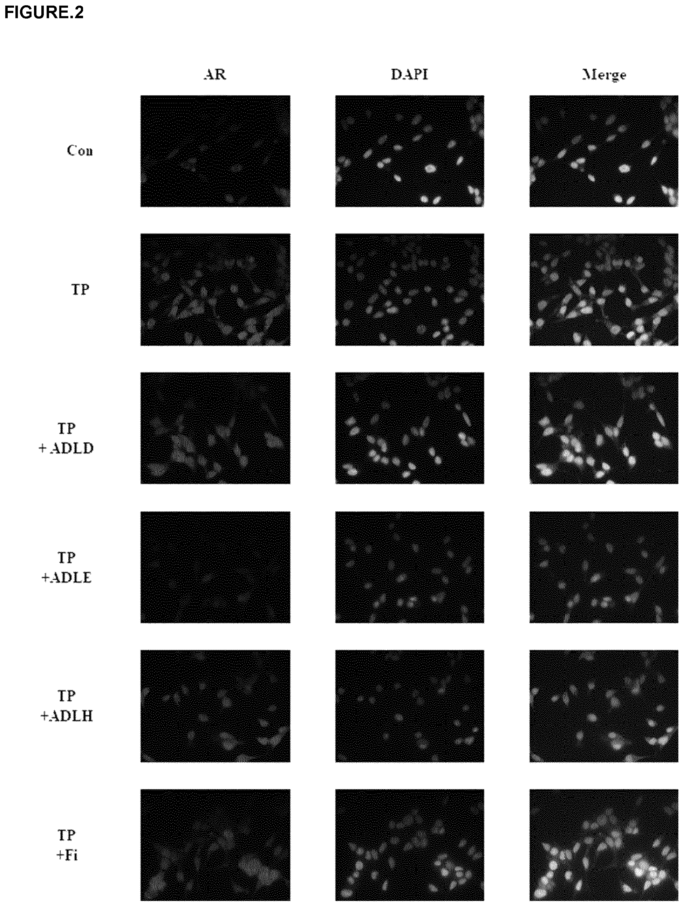

As shown in FIG. 2, it was confirmed that a significantly excellent AR inhibitory effect is shown in the *Abeliophyllum distichum* leaf ethanol extract (ADLE).

### Example 4. Confirmation of expression of androgen signaling-related factors according to Abeliophyllum distichum harvest period

The difference in expression levels between AR and PSA induced by testosterone according to the harvest time of *Abeliophyllum distichum* leaves was confirmed. Leaves harvested in late spring were harvested in mid-May, and leaves harvested in late autumn were harvested in mid-September, and extracts were prepared according to the ethanol extraction method of Example 1-2. Meanwhile, the androgen signaling-related factors were identified by the same method as in Example 2.

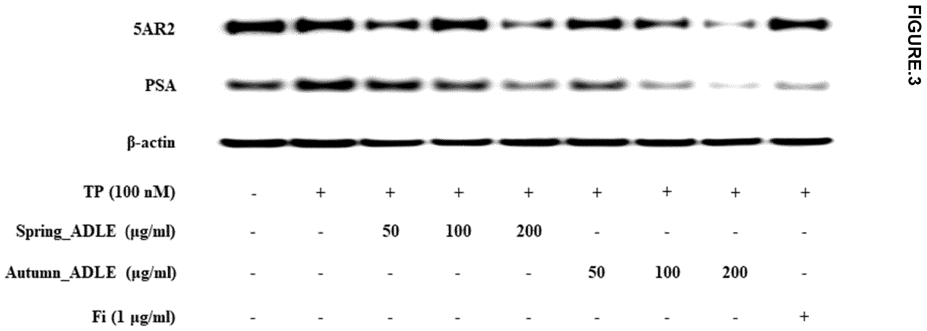

As shown in FIG. 3, it was confirmed that the inhibitory effect on 5AR and PSA expression in the late autumn-harvested *Abeliophyllum distichum* leaf ethanol extract is significantly superior to that obtained with *Abeliophyllum distichum* leaves harvested in late spring.

### Example 5. Identification of components of Abeliophyllum distichum leaf ethanol extract

### 5-1. Result of component analysis

For the component analysis of the *Abeliophyllum distichum* leaf ethanol extract, UPLC-MS was used.

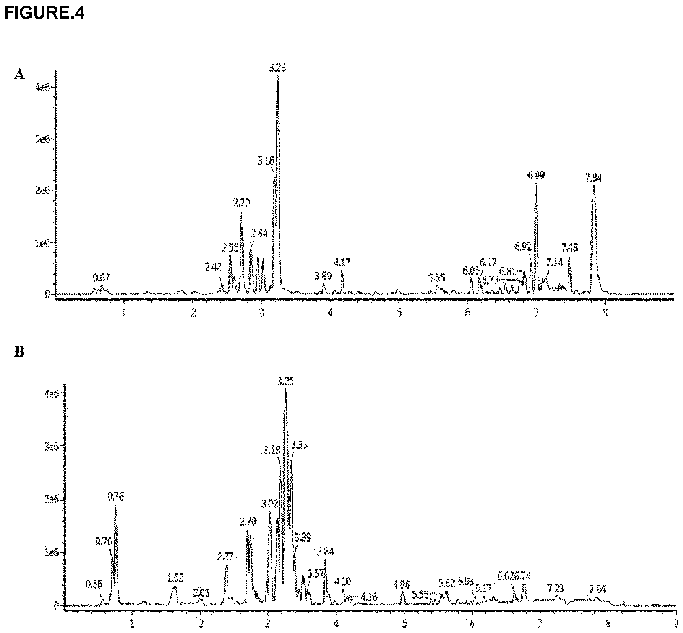

The result is shown in FIG. 4 and Table 1.

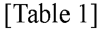

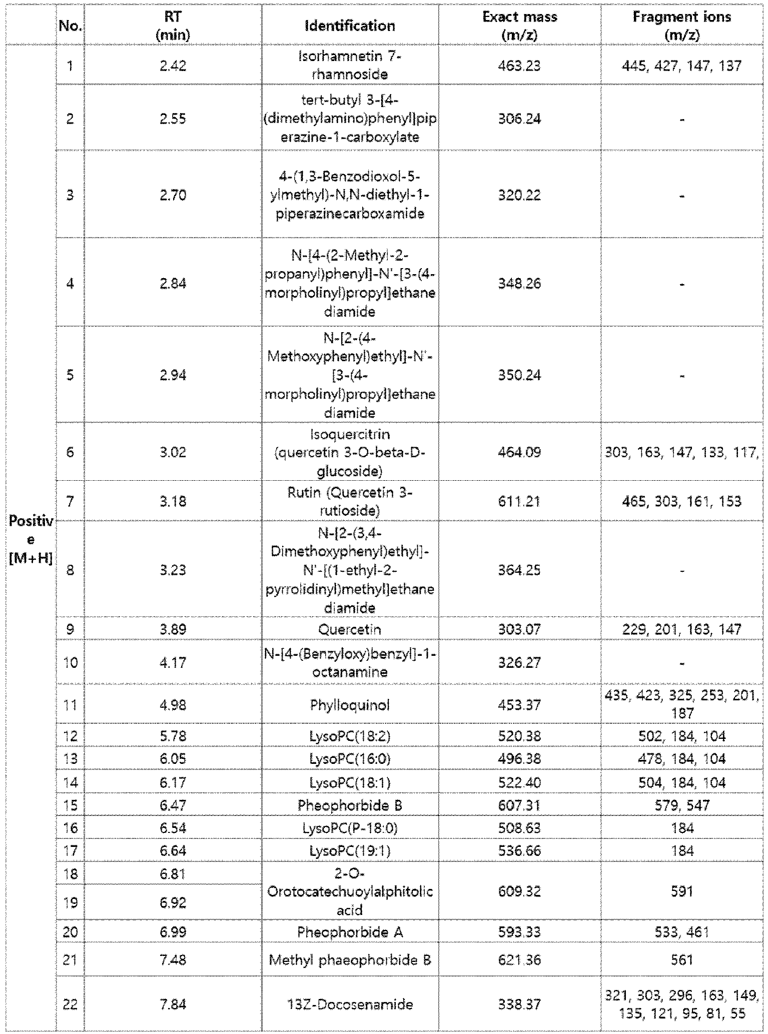

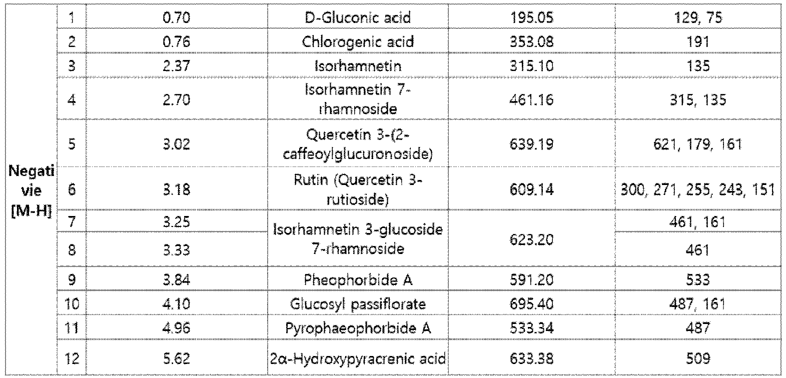

Based on the result, as the functional ingredients of the *Abeliophyllum distichum* leaf ethanol extract, isoquercetin (IQ), rutin, chlorogenic acid (CA), and isorhamnetin 3-glucoside-7-rhamnoside (IR) were selected, and their effects on the expression of BPH-related factors were confirmed.

### 5-2. Confirmation of effect of active ingredient of Abeliophyllum distichum leaf ethanol extract

A difference in testosterone-induced AR, 5AR2, and PSA expression levels of four types (IQ, Rutin, CA, and IR) of the functional ingredient candidates selected in Example 5-1 was confirmed. The method was performed in the same manner as in Example 2.

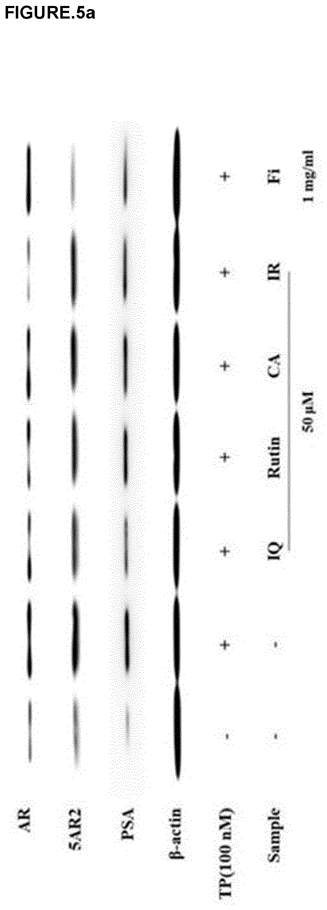

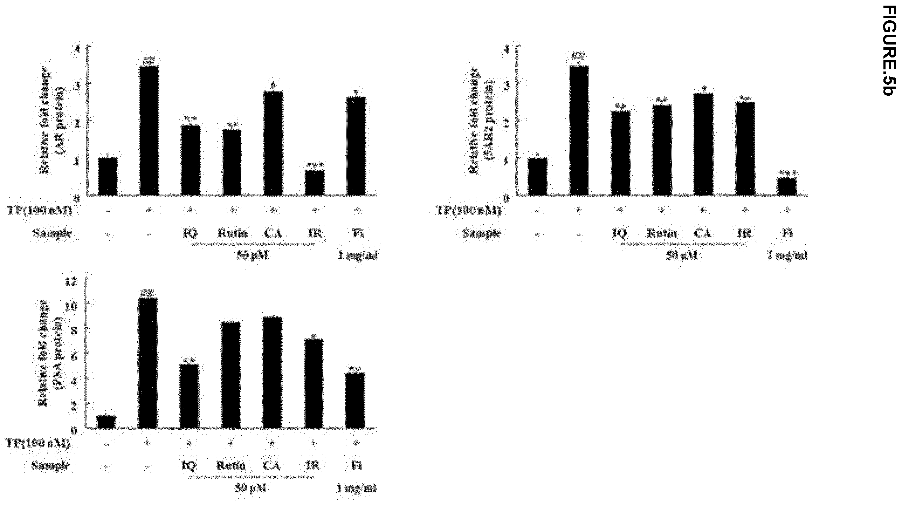

As shown in FIGS. 5A and 5B, it was confirmed that, among the four candidates, isoquercetin exhibited the most excellent effect.

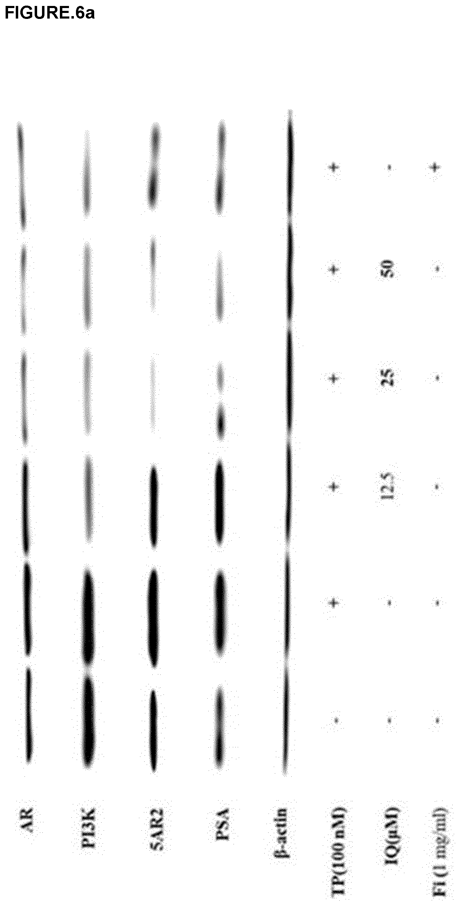

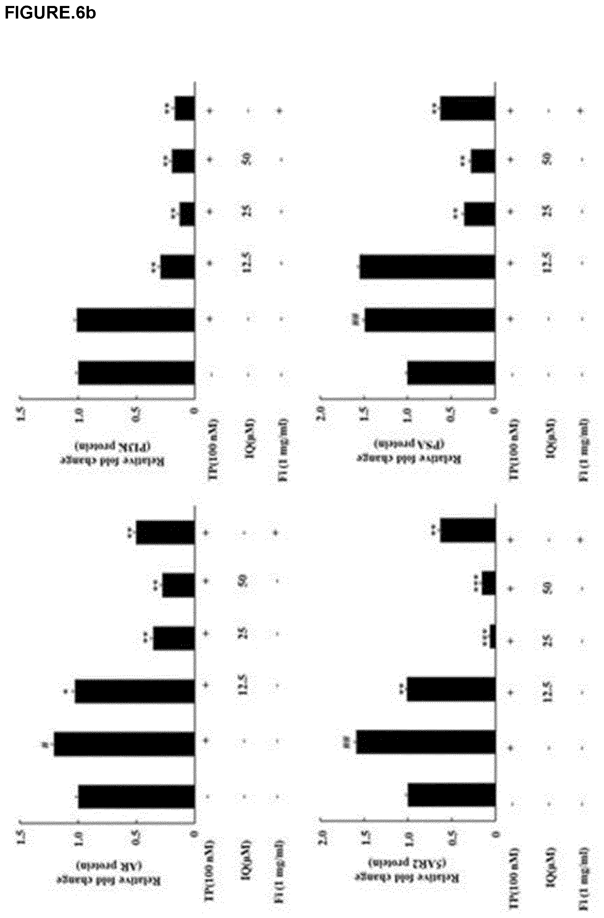

Moreover, as shown in FIGS. 6A and 6B, it was confirmed that the expression of AR, PI3K, and 5AR2 was significantly reduced depending on the concentration of isoquercetin.

### 5-3. Confirmation of expression of androgen signaling-related factors of isoquercetin (IQ)

BPH-1 cells were treated with testosterone (100 nM) to induce cell proliferation, and then treated with each of 25, 50, and 100 µM isoquercetin (IQ), and 25, 50, and 100 µg/mL of the *Abeliophyllum distichum* leaf ethanol extract for 24 hours to confirm inhibitory effects on the expression of PI3K, and cell proliferation factors PCNA and cyclin D1. This method was performed in the same manner as in Example 2. PCNA is a factor that is increased when BPH occurs, and cyclin D1 corresponds to a cell cycle upregulator in prostatic cells.

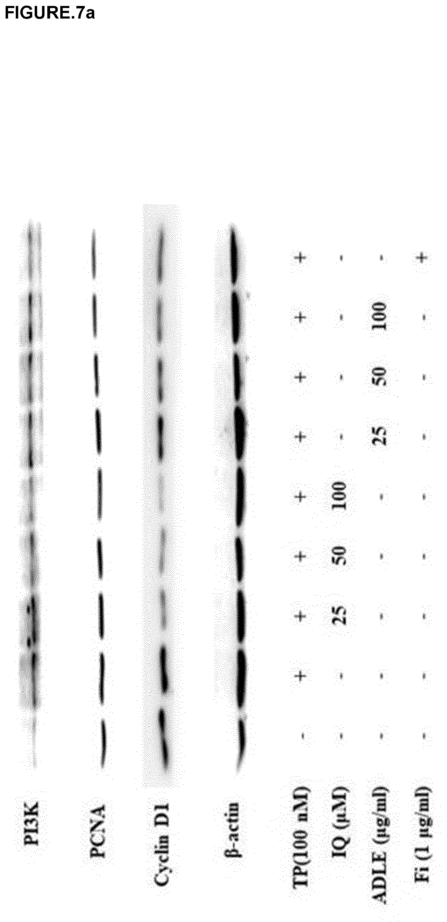

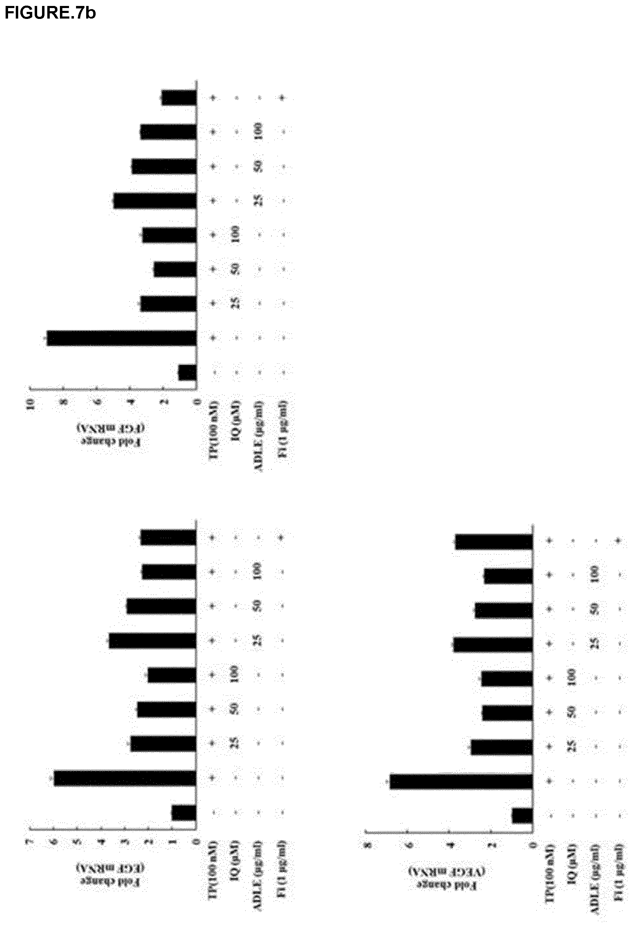

As shown in FIGS. 7A and 7B, it was confirmed that PI3K, PCNA and cyclin D1 expression was significantly reduced depending on the concentrations of isoquercetin and the *Abeliophyllum distichum* leaf ethanol extract.

### Example 6. Confirmation of isoquercetin content according to Abeliophyllum distichum extraction period

To analyze isoquercetin, which is an active ingredient, according to the extraction period of *Abeliophyllum distichum* leaves, UPLC-MS was used.

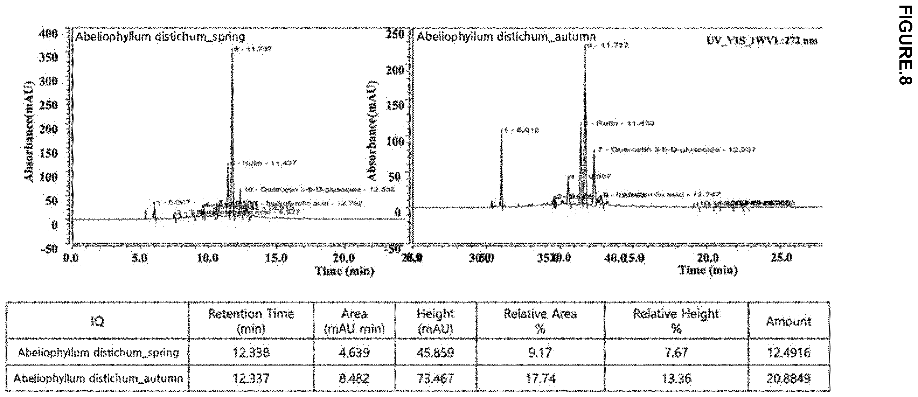

As shown in FIG. 8, it was confirmed that the autumn content (20.8849 mg/g) is higher than the spring content (12.4916 mg/g).

That is, it was determined that, as a functional ingredient of *Abeliophyllum distichum,* isoquercetin exhibiting both of functionality and time-dependent differentiation is appropriate.

### Example 7. Confirmation of effect on BPH animal model

### 7-1. Confirmation of BPH treatment effect of Abeliophyllum distichum extract

To confirm whether an *Abeliophyllum distichum* extract is also effective *in vivo,* BPH animal models were manufactured to conduct experiments (see FIG. 9).

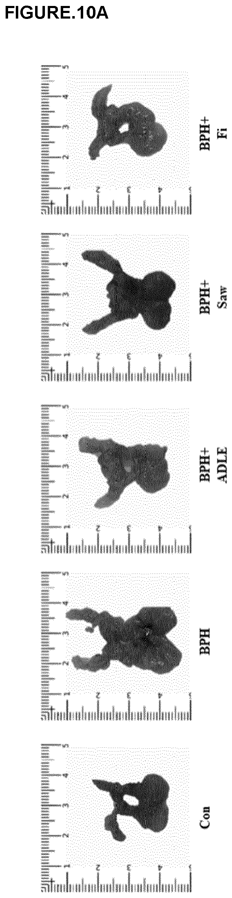

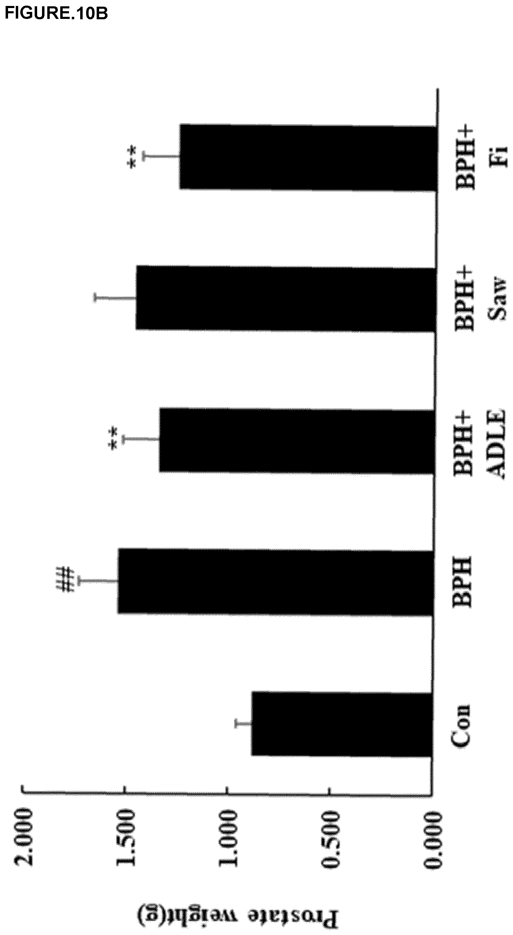

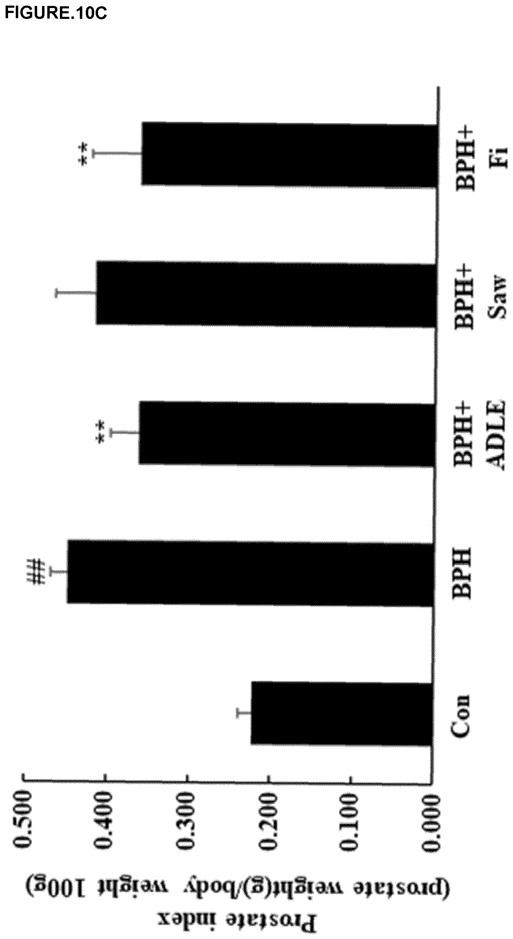

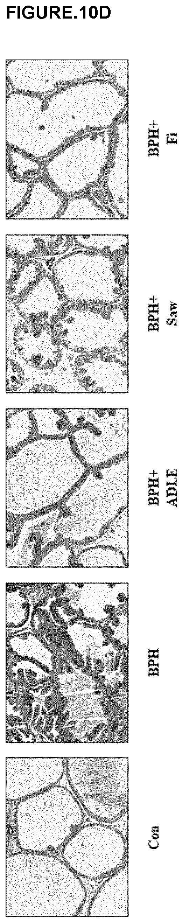

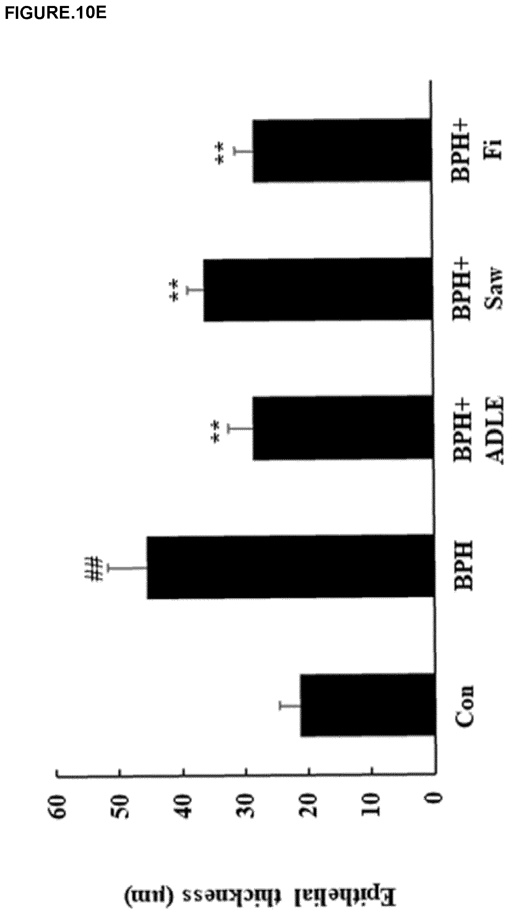

As shown in FIGS. 10A to 10E, it was confirmed that mice fed the autumn-harvested *Abeliophyllum distichum* leaf extract (ADLE) had a significantly lower prostate index than a positive control. Specifically, in the ADLE-administered group, a prostate weight was significantly reduced, and a ratio of the prostate weight to a body weight was also significantly reduced compared to the BPH-induced model, the prostate epithelial thickness was also significantly reduced compared to that of the BPH model, and compared to the positive controls, saw palmetto and finasteride, the ADLE effect is superior.

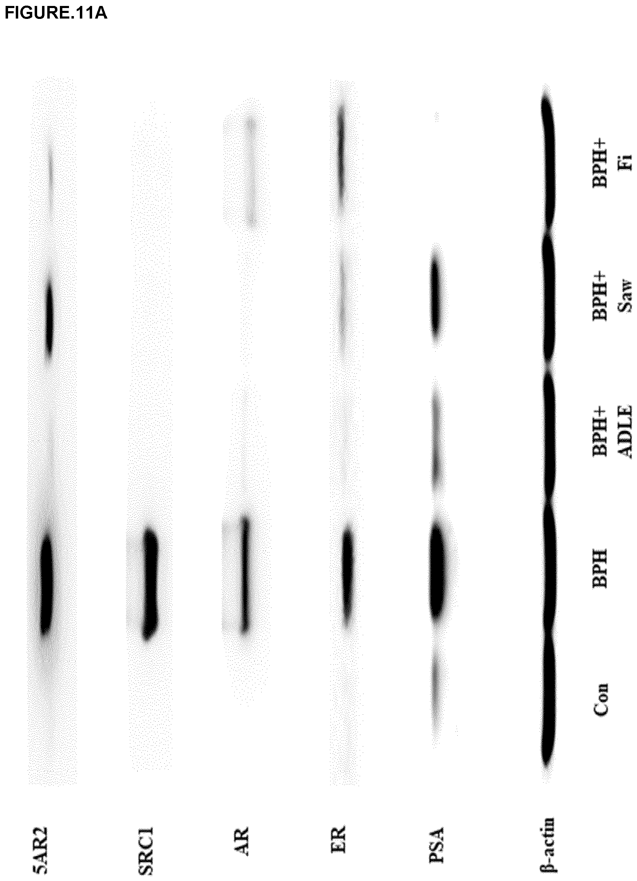

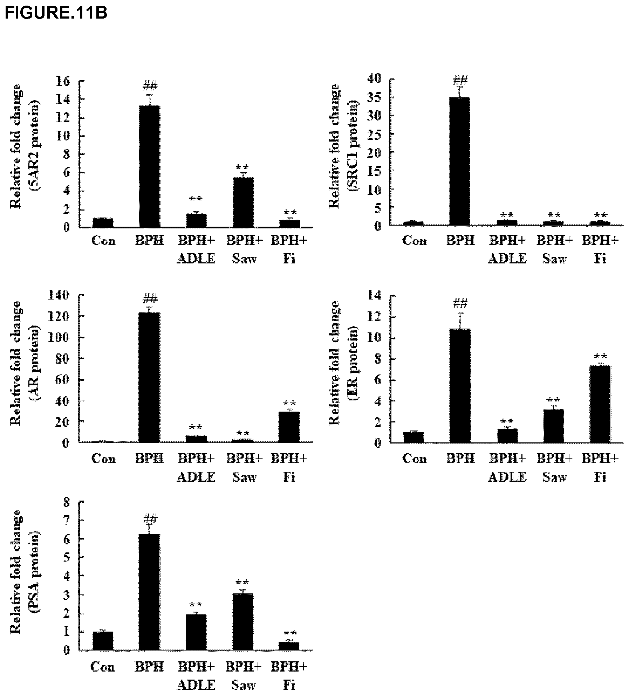

In addition, as shown in FIGS. 11A and 11B, it was confirmed that the expression levels of 5AR2, SRC1, AR, ER, and PSA in prostate tissue were significantly reduced by ADLE intake.

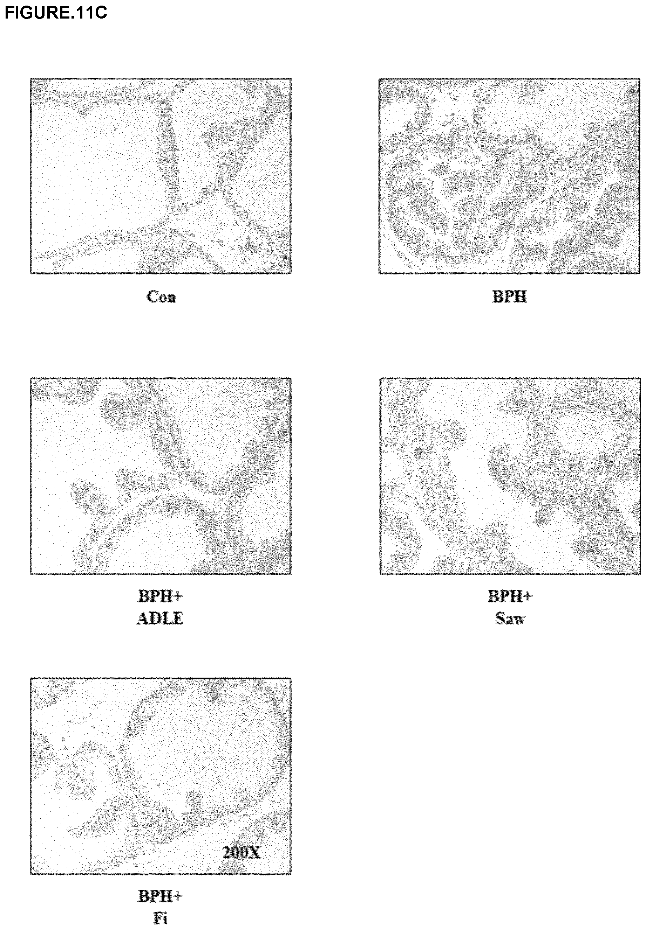

In addition, as shown in FIG. 11C, as a result of H&E staining of prostate tissue and observation of prostate epithelial cells, it was confirmed that, compared to the control (BPH), in the ADLE-fed group, the thickness of epithelial cells was significantly reduced to a CON level. This is a similar or excellent result compared to those of the positive controls, saw palmetto and finasteride.

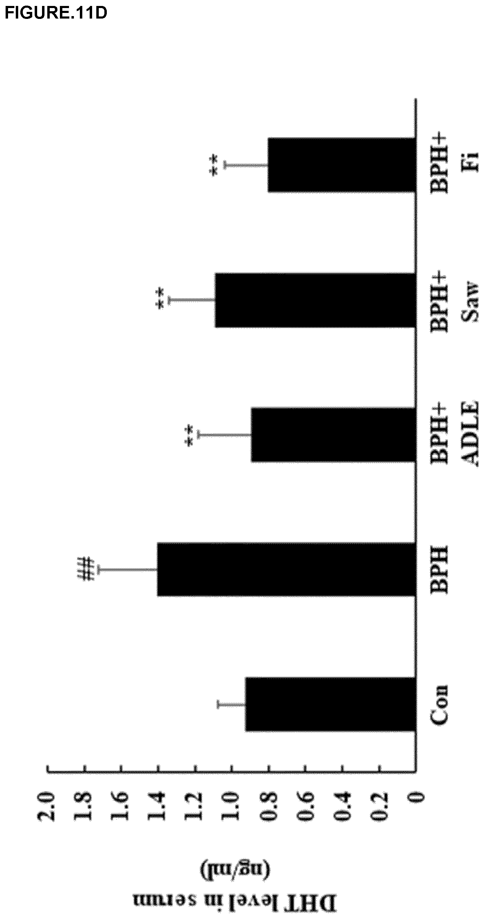

As shown in FIG. 11D, it was also confirmed that the DHT level in serum was significantly reduced, which is a result that supports the excellent therapeutic effect of BPH caused by reducing a DHT level causing the increase in prostate growth and enlargement.

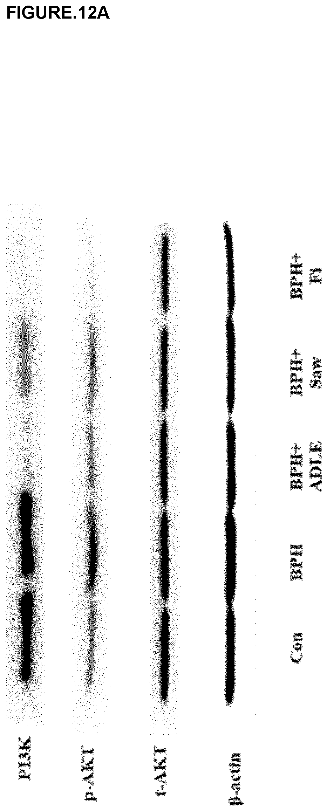

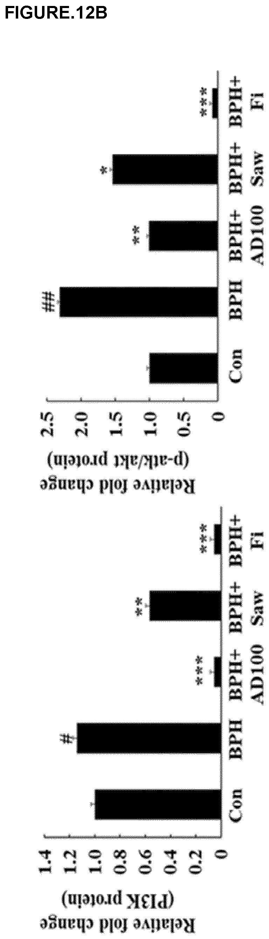

As shown in FIGS. 12A and 12B, it was confirmed that the expression levels of PI3K and p-AKT in prostate tissue are significantly reduced by ADLE intake.

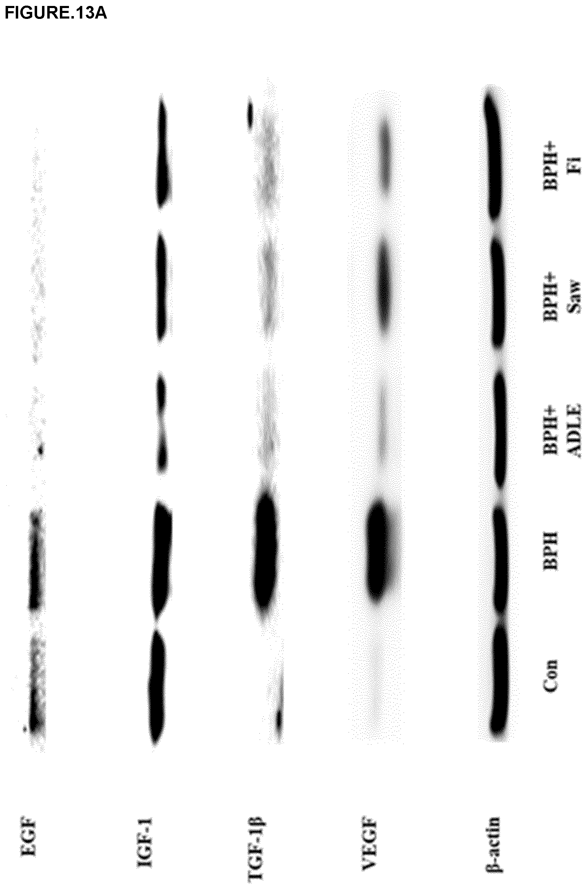

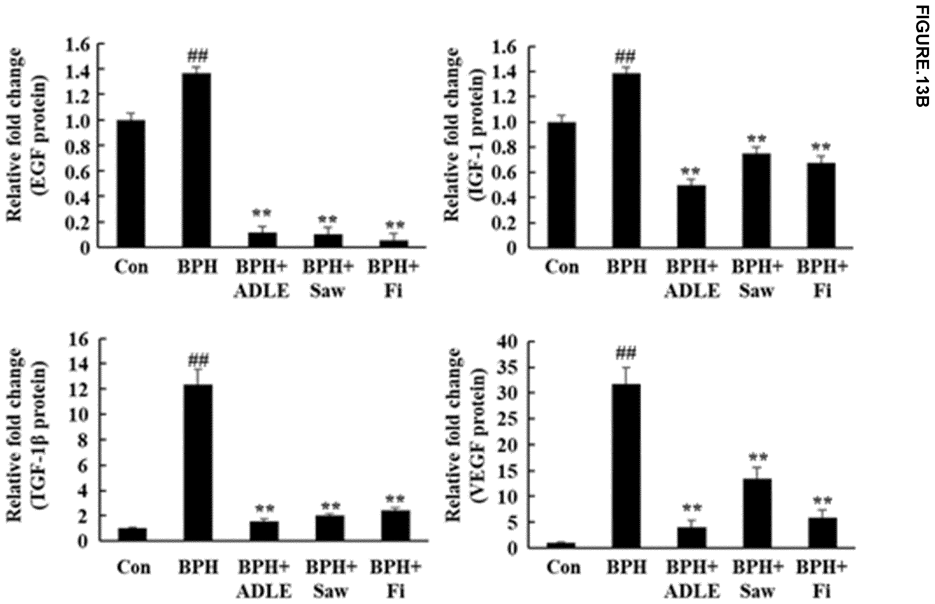

As shown in FIGS. 13A and 13B, it was confirmed that the expression levels of EGF, IGF-1, TGF-1β, and VEGF in prostate tissue were significantly reduced by ADLE intake.

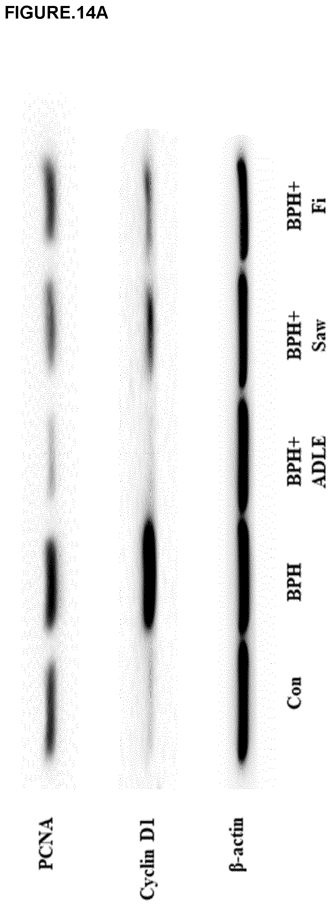

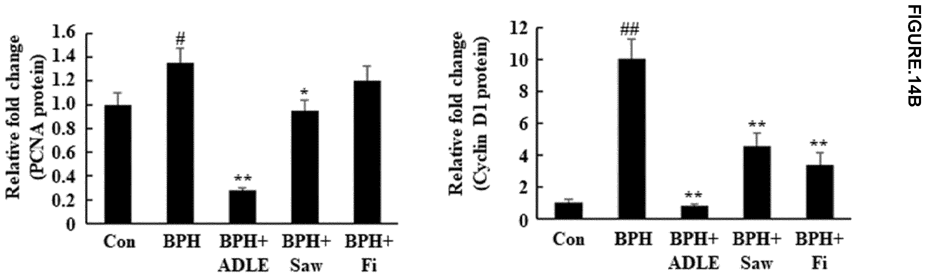

As shown in FIGS. 14A and 14B, it was confirmed that the expression levels of PCNA and cyclin D1 in prostate tissue were significantly reduced by ADLE intake.

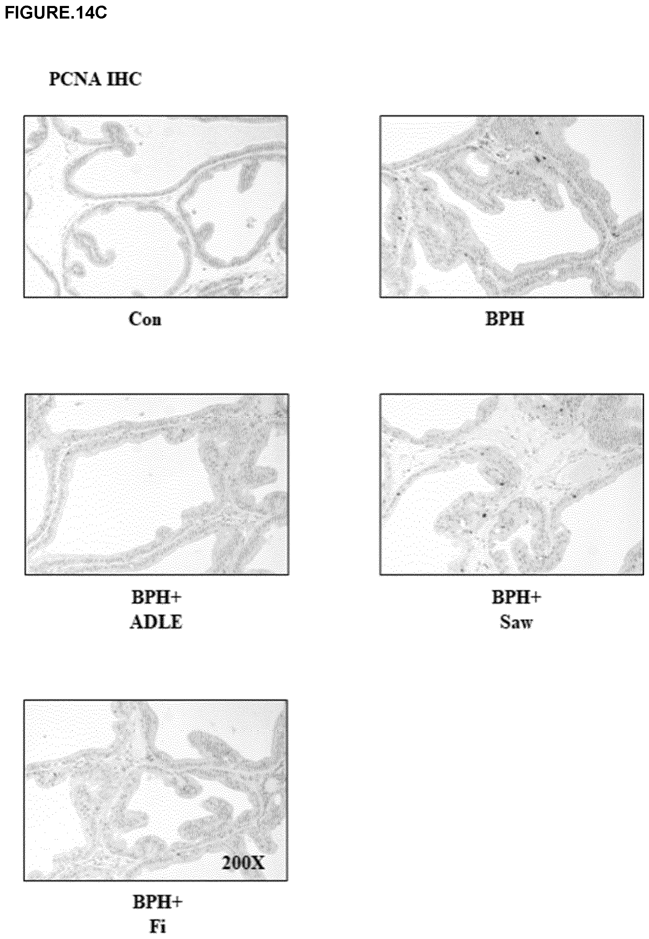

As shown in FIG. 14C, it was confirmed that the expression level of PCNA in prostate tissue was significantly reduced by ADLE intake.

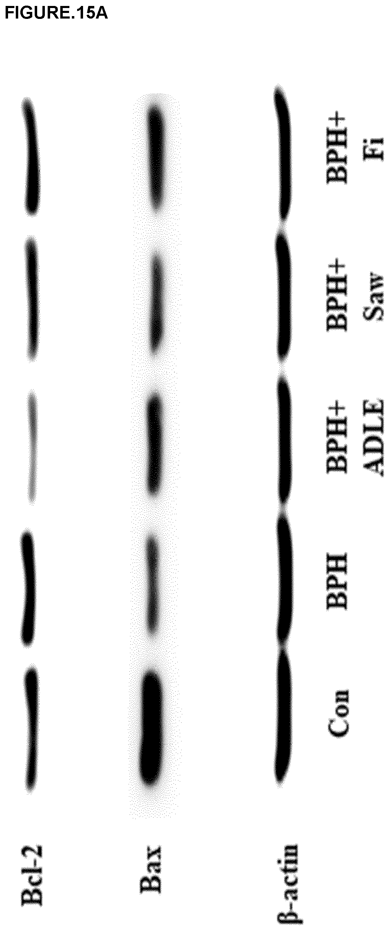

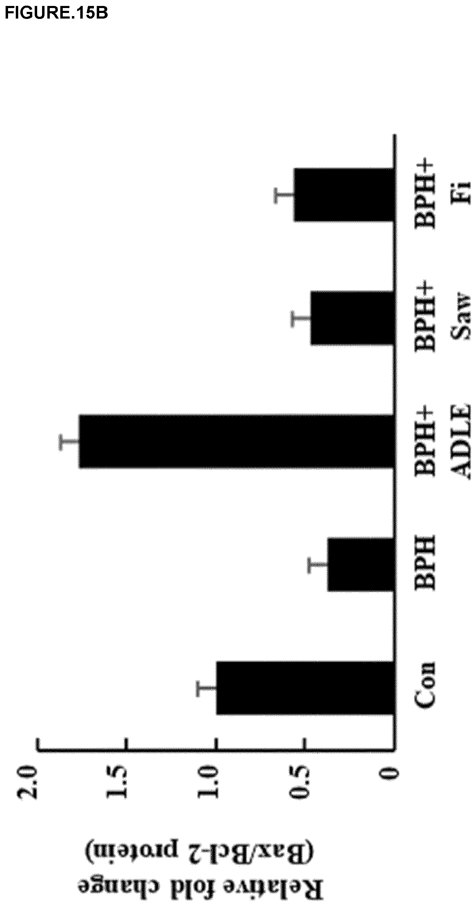

As shown in FIGS. 15A and 15B, it was confirmed that the expression level of Bcl-2 in prostate tissue was significantly reduced by ADLE intake, and the expression level of Bax was significantly increased by ADLE intake.

Based on all of the above examples, it was confirmed that an *Abeliophyllum distichum* extract not only significantly improves levels of androgen receptors and prostate-related factors, for example, reduces the expression levels of androgenic receptor signaling-related factors, 5AR2, SRC1, AR, ER, and PSA, but also exhibits a significant therapeutic effect on BPH animal models. Therefore, it can be seen that the *Abeliophyllum distichum* extract of the present invention can be used in therapeutic agents for androgenic receptor- and prostate-related diseases.

### 7-2. Confirmation of BPH treatment effect of isoquercetin

In addition to Example 7-1, to confirm whether isoquercetin, which is the functional ingredient of the *Abeliophyllum distichum* extract, is effective *in vivo,* BPH animal models were manufactured to conduct experiments.

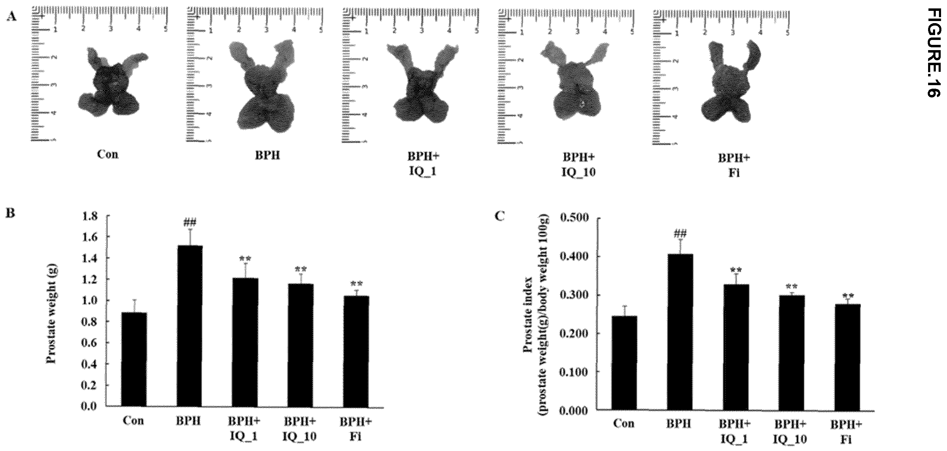

As shown in FIG. 16, as a result of oral administration of isoquercetin at a low concentration (1 mg/kg) and a high concentration (10 mg/kg), it was confirmed that a prostate index was lowered depending on an isoquercetin concentration. Specifically, in the isoquercetin-administered group, a prostate weight was significantly reduced, and a ratio of the prostate weight to a body weight was also significantly decreased compared to the BPH-induced model.

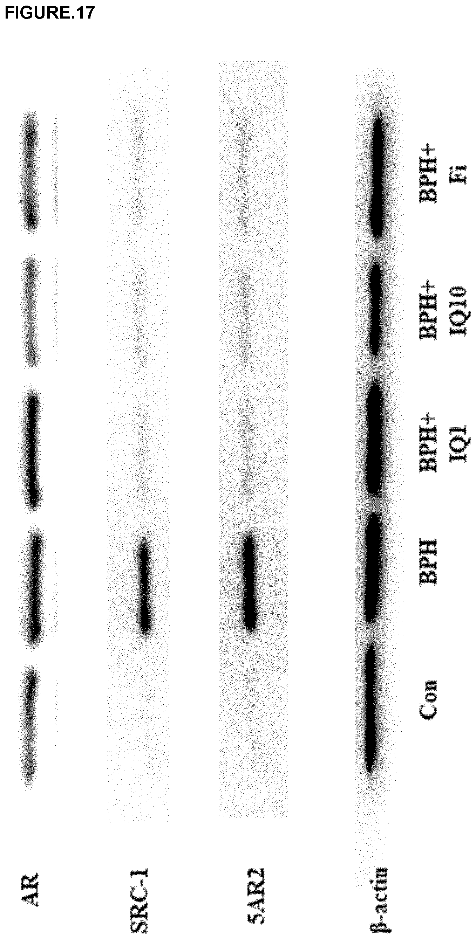

As shown in FIG. 17, it was confirmed that the expression levels of AR, SRC-1 and 5AR2 in prostate tissue were reduced depending on an isoquercetin intake concentration.

Therefore, it was confirmed that isoquercetin not only significantly improves levels of androgen receptors and prostate-related factors, for example, reduces the expression levels of androgenic receptor signaling-related factors, 5AR2, SRC1, AR, ER, and PSA, but also exhibits a significant therapeutic effect on BPH animal models. Thus, it can be seen that isoquercetin, which is the functional ingredient of the *Abeliophyllum distichum* extract, can be used in therapeutic agents for androgenic receptor- and prostate-related diseases.

It should be understood by those of ordinary skill in the art that the above description of the present invention is exemplary, and the exemplary embodiments disclosed herein can be easily modified into other specific forms without departing from the technical spirit or essential features of the present invention. Therefore, the exemplary embodiments described above should be interpreted as illustrative and not restrictive in any aspect.

### [Industrial Applicability]

Since an *Abeliophyllum distichum* extract or isoquercetin according to the present invention has androgenic receptor inhibitory activity, and an excellent therapeutic effect on a benign prostatic hyperplasia (BPH) animal model, it can be widely applied in androgenic receptor-related diseases such as BPH, androgen-dependent hair loss, dysuria and prostate cancer, and thus has industrial applicability.

## Claims

1. A pharmaceutical composition for preventing or treating an androgenic receptor-related disease, comprising isoquercetin or an *Abeliophyllum distichum* extract as an active ingredient.

2. The pharmaceutical composition of claim 1, wherein the extract is an extract that is prepared with one or more solvents selected from the group consisting of water, C₁ to C₆ alcohols, acetone, ether, benzene, chloroform, ethyl acetate, methylene chloride, hexane, cyclohexane, petroleum ether, a subcritical fluid, and a supercritical fluid.

3. The pharmaceutical composition of claim 1, wherein the androgenic receptor-related disease is one or more selected from the group consisting of prostate cancer, benign prostatic hyperplasia (BPH), dysuria, and hair loss.

4. The pharmaceutical composition of claim 1, wherein the *Abeliophyllum distichum* extract comprises isoquercetin.

5. The pharmaceutical composition of claim 1, wherein the *Abeliophyllum distichum* extract is an *Abeliophyllum distichum* leaf extract.

6. The pharmaceutical composition of claim 1, wherein the composition inhibits the expression of an androgenic receptor.

7. The pharmaceutical composition of claim 1, wherein the composition inhibits one or more androgen signaling-related factors selected from the group consisting of 5α-reductase 2 (5AR2), steroid receptor coactivator 1 (SRC1), estrogen receptor (ER), and prostate-specific antigen (PSA).

8. The pharmaceutical composition of claim 1, wherein the composition inhibits a PI3K/AKT signaling pathway.

9. A food composition for preventing or alleviating an androgenic receptor-related disease, comprising isoquercetin or an *Abeliophyllum distichum* extract as an active ingredient.

10. The food composition of claim 9, wherein the food composition is a health functional food.

11. The food composition of claim 9, wherein the androgenic receptor-related disease is one or more selected from the group consisting of prostate cancer, benign prostatic hyperplasia (BPH), dysuria, and hair loss.

12. A method of preventing or treating an androgenic receptor-related disease, comprising administering a composition comprising isoquercetin or an *Abeliophyllum distichum* extract as an active ingredient to a subject.

13. A use of a composition comprising isoquercetin or an *Abeliophyllum distichum* extract as an active ingredient for preventing or treating an androgenic receptor-related disease.

14. A use of isoquercetin or an *Abeliophyllum distichum* extract for producing a drug for preventing or treating an androgenic receptor-related disease.
